# EUROPEAN PATENT SPECIFICATION

(11) **EP 3 259 369 B1**
(45) Date of publication and mention of the grant of the patent: **27.04.2022**
(21) Application number: 16706330.4
(22) Date of filing: 17.02.2016
(51) Int. Cl.: C12Q 1/6886

(54) **METHODS AND KITS FOR THE MOLECULAR SUBTYPING OF BLADDER CANCER**
VERFAHREN UND KITS ZUR MOLEKULAREN SUBTYPISIERUNG VON BLASENKARZINOM
PROCÉDÉS ET KITS DE SOUS-TYPAGE MOLÉCULAIRE DU CANCER DE LA VESSIE

(30) Priority: 17.02.2015 WO PCT/EP2015/053283
(43) Date of publication of application: 27.12.2017
(73) Proprietor: BioNTech Diagnostics GmbH, 55131 Mainz (DE); STRATIFYER Molecular Pathology GmbH, 50935 Köln (DE)
(72) Inventor: WIRTZ, Ralph Markus, 50677 Köln (DE)
(74) Representative: Michalski Hüttermann & Partner Patentanwälte mbB
(86) International application number: PCT/EP2016/053372
(87) International publication number: WO 2016/131875

(56) References cited:
- WO-A1-2014/085666
- WO-A1-2015/024942
- CHOI WOONYOUNG ET AL: "Identification of Distinct Basal and Luminal Subtypes of Muscle-Invasive Bladder Cancer with Different Sensitivities to Frontline Chemotherapy", CANCER CELL, vol. 25, no. 2, 10 February 2014 (2014-02-10), pages 152-165, XP028610273, ISSN: 1535-6108, DOI: 10.1016/J.CCR.2014.01.009
- J. S. DAMRAUER ET AL: "Intrinsic subtypes of high-grade bladder cancer reflect the hallmarks of breast cancer biology", PROCEEDINGS OF THE NATIONAL ACADEMY OF SCIENCES, vol. 111, no. 8, 11 February 2014 (2014-02-11), pages 3110-3115, XP055269416, US ISSN: 0027-8424, DOI: 10.1073/pnas.1318376111
- WOONYOUNG CHOI ET AL: "Intrinsic basal and luminal subtypes of muscle-invasive bladder cancer", NATURE REVIEWS. UROLOGY, vol. 11, no. 7, 24 June 2014 (2014-06-24), pages 400-410, XP055269413, US ISSN: 1759-4812, DOI: 10.1038/nrurol.2014.129
- MARGARET A. KNOWLES ET AL: "Molecular biology of bladder cancer: new insights into pathogenesis and clinical diversity", NATURE REVIEWS. CANCER, vol. 15, no. 1, 23 December 2014 (2014-12-23), pages 25-41, XP055269452, GB ISSN: 1474-175X, DOI: 10.1038/nrc3817
- WIRTZ R M ET AL: "Molecular classification of bladder cancer", PATHOLOGE, BERLIN, DE, vol. 37, no. 1, 15 January 2016 (2016-01-15), pages 52-60, XP035627362, ISSN: 0172-8113, DOI: 10.1007/S00292-015-0134-8 [retrieved on 2016-01-15]
- ZHAO JUNJIE ET AL: "Prognostic role of HER2 expression in bladder cancer: a systematic review and meta-analysis", INTERNATIONAL UROLOGY AND NEPHROLOGY, AKADEMIAI, BUDAPEST, HU, vol. 47, no. 1, 11 November 2014 (2014-11-11), pages 87-94, XP035418517, ISSN: 0301-1623, DOI: 10.1007/S11255-014-0866-Z [retrieved on 2014-11-11]
- SURENDRA B KOLLA ET AL: "Prognostic significance of Her2/neu overexpression in patients with muscle invasive urinary bladder cancer treated with radical cystectomy", INTERNATIONAL UROLOGY AND NEPHROLOGY, KLUWER ACADEMIC PUBLISHERS, DO, vol. 40, no. 2, 26 September 2007 (2007-09-26), pages 321-327, XP019616319, ISSN: 1573-2584
- Stefan Kruger ET AL: "Overexpression of c-erbB-2 oncoprotein in muscle-invasive bladder carcinoma: relationship with gene amplification, clinicopathological parameters and prognostic outcome", International journal of oncology, 1 November 2002 (2002-11-01), XP055676801, GR ISSN: 1019-6439, DOI: 10.3892/ijo.21.5.981

### SEQUENCE LISTING

<110> BioNTech AG STRATIFYER Molecular Pathology GmbH
<120> Methods and kits for the molecular subtyping of bladder cancer
<130> 674-145 PCT
<150> PCT/EP2015/053283
   <151> 2015-02-17
<160> 21
<170> PatentIn version 3.5
<210> 1
   <211> 19
   <212> DNA
   <213> Artificial Sequence
<220>
   <223> ESR1_F
<400> 1
   agagggtgcc aggctttgt 19
<210> 2
   <211> 22
   <212> DNA
   <213> Artificial Sequence
<220>
   <223> ESR1_R
<400> 2
   aggatctcta gccaggcaca tt 22
<210> 3
   <211> 27
   <212> DNA
   <213> Artificial Sequence
<220>
   <223> ESR1_P
<400> 3
   tttgaccctc catgatcagg tccacct 27
<210> 4
   <211> 21
   <212> DNA
   <213> Artificial Sequence
<220>
   <223> HER2_F
<400> 4
   gaactcacct acctgcccac c 21
   <210> 5
   <211> 21
   <212> DNA
   <213> Artificial Sequence
<220>
   <223> HER2_R
<400> 5
   gacctgcctc acttggttgt g 21
<210> 6
   <211> 22
   <212> DNA
   <213> Artificial Sequence
<220>
   <223> HER2_P
<400> 6
   ccaggaggtg cagggctacg tg 22
<210> 7
   <211> 22
   <212> DNA
   <213> Artificial Sequence
<220>
   <223> KI67_F
<400> 7
   cgagacgcct ggttactatc aa 22
<210> 8
   <211> 25
   <212> DNA
   <213> Artificial Sequence
<220>
   <223> KI67_R
<400> 8
   ggatacggat gtcacattca atacc 25
<210> 9
   <211> 23
   <212> DNA
   <213> Artificial Sequence
<220>
   <223> KI67_P
<400> 9
   acggtcccca ctttcccctg agc 23
<210> 10
   <211> 27
   <212> DNA
   <213> Artificial Sequence
<220>
   <223> PGR_F
<400> 10
   aaacttcttg ataacttgca tgatctt 27
<210> 11
   <211> 25
   <212> DNA
   <213> Artificial Sequence
<220>
   <223> PGR_R
<400> 11
   caataacttc agacatcatt tctgg 25
<210> 12
   <211> 30
   <212> DNA
   <213> Artificial Sequence
<220>
   <223> PGR_P
<400> 12
   cgggactgga taaatgtatt caagcagtac 30
<210> 13
   <211> 22
   <212> DNA
   <213> Artificial Sequence
<220>
   <223> RAC_F
<400> 13
   gaatgtgcgg aatctgtttg ag 22
<210> 14
   <211> 21
   <212> DNA
   <213> Artificial Sequence
<220>
   <223> RAC_R
<400> 14
   tcgccaactg gataaattgg a 21
<210> 15
   <211> 23
   <212> DNA
   <213> Artificial Sequence
<220>
   <223> RAC_P
<400> 15
   actgagaatc tccacccggc gca 23
<210> 16
   <211> 20
   <212> DNA
   <213> Artificial Sequence
<220>
   <223> B2M_F
<400> 16
   gtatgcctgc cgtgtgaacc 20
<210> 17
   <211> 22
   <212> DNA
   <213> Artificial Sequence
<220>
   <223> B2M_R
<400> 17
   ggcatcttca aacctccatg at 22
<210> 18
   <211> 25
   <212> DNA
   <213> Artificial Sequence
<220>
   <223> B2M_P
<400> 18
   agtgggatcg agacatgtaa gcagc 25
<210> 19
   <211> 19
   <212> DNA
   <213> Artificial Sequence
<220>
   <223> CALM2_F
<400> 19
   aggaggcgaa ttagtccga 19
<210> 20
   <211> 20
   <212> DNA
   <213> Artificial Sequence
<220>
   <223> CALM2_R
<400> 20
   gctcttcagt cagttggtca 20
<210> 21
   <211> 22
   <212> DNA
   <213> Artificial Sequence
<220>
   <223> CALM2_P
<400> 21
   tcgcgtctcg gaaaccggta gc 22

## Description

The present invention relates to *in vitro* methods of identifying a molecular subtype of a tumor in a patient having bladder cancer and to methods of stratifying a bladder cancer patient for tumor treatment. It further relates to corresponding kits and their uses, as well as to prognostic biomarkers for bladder cancer, in particular singular prognostic biomarkers.

### BACKGROUND OF THE INVENTION

Based on histopathological evaluations bladder cancer is believed to be very heterogeneous and to comprise a whole spectrum of distinct histopathological subtypes (papillary, non-papillary, squamous cell carcinoma, adenocarcinoma, sarcoma, small cell carcinoma, etc.). It is also known that bladder cancers have widely variable clinical outcomes and respond markedly different to loco-regional or systemic treatments. Of great importance for prognosis and treatment of bladder cancer is the invasion depth.

Tumors that have not entered into the muscle layer are called superficial or non-muscle-invasive tumors (non-muscle-invasive bladder cancer, NMIBC). NMIBCs account for approximately 70% of all bladder tumors. NMIBC is not immediately life-threatening, but has a high propensity for recurrence that necessitates lifelong and costly surveillance. Currently, more than 30% of superficial bladder cancer patients are clearly undertreated by the current treatment standards (transurethral resection and Bacillus Calmette-Guerin (BCG)-instillation), develop metastasis and die, despite the fact that there were additional treatment options in the initial curative treatment situation. Superficial bladder tumors can be shaved off by resectoscopes as part of transurethral resections, which also serves for the pathological staging of the tumor. Treatment by intravesicular delivery of BCG, an immunotherapeutic approach, is used to prevent recurrence of superficial bladder cancer. However, up to 60 % of NMIBCs do recur and need further and more radical surgery (i.e., cystectomy), loco-regional treatment (e.g., radiation) and/or chemotherapy (which may be directly instilled into the bladder). More particularly, a "rule of 30's" has been observed, meaning that 30% of all patients stay free of recurrence after local resection and adjuvant instillation therapy with BCG, 30% do recur and require more radical therapy, while 30% rapidly progress and become metastatic. Therefore, while for many cases the local resection and BCG treatment is adequate and the bladder can be preserved for a substantial time, other patients are harmed by these current treatment options and would require an immediate radical cystectomy and perioperative chemotherapy to enable curative treatment. Apparently, there is a great unmet clinical need to better assess the individual risk of non-muscle-invasive bladder cancer patients to make better use of the existing treatment options in the initial curative setting, e.g., by stratifying patients for more individualized treatment approaches.

Tumors that have invaded further to enter into the muscle layer are called muscle-invasive bladder cancers (MIBCs). MIBCs require radical cystectomy with perioperative cisplatin-based combination chemotherapy. Currently, the treatment selection is solely based on clinicopathological characteristics that are woefully inaccurate, thereby resulting in an unacceptably high rate of clinical understaging and consequently inadequate and, thus, life-threatening treatment. Moreover, standard chemotherapy is only effective in 30% of all cases. Importantly, neoadjuvant treatment using platinum-based chemotherapy offers the opportunity to achieve pathologically complete responses of muscle-invasive bladder cancer patients, which is then associated with excellent long-term survival and less radical surgical interventions. However, for tumors not responding to neoadjuvant chemotherapy, an immediate radical surgical intervention seems to be the better treatment option to prevent further progress of the cancer and death. Therefore, there is a great unmet clinical need to identify the tumors that are likely to respond to chemotherapy and to decide on the optimal treatment procedure (neoadjuvant or adjuvant chemotherapy). In addition, targeted therapy options such as anti-hormonal (endocrine) or anti-HER2 therapies are currently not being applied, nor has it been possible to establish the detection of these receptors by conventional methods to enable prediction of the response to respective targeted treatment approaches.

In conclusion, the medical need to better classify bladder cancer patients for their individual risk of recurrence and response to chemotherapy or alternative treatment options is high for both the non-muscle-invasive and muscle-invasive situation. Importantly, for the very heterogeneous groups of both bladder cancer stages the optimal therapeutic procedure is not clear as the prognosis cannot be reliably assessed by current clinicopathological features.

International patent application WO 2014/085666 discloses a method of detecting and characterizing high-risk bladder cancers, in which primary tumors, circulating tumor cells, serum, and urine are tested for the level of expression or activation of a plurality of genes. Damrauer, J.S. et al. (Proc Natl Acad Sci U S A. (2014) 111(8), 3110-3115) relates to identifying intrinsic molecular subtypes of high-grade bladder cancer.

The current worldwide applied standard methodology for the detection of the receptor status of cancers, e.g., breast cancers, is immunohistochemistry (IHC) from formalin-fixed and paraffin-embedded (FFPE) biopsy or resection tissue. In breast cancer, administration of endocrine or targeted systemic treatment (e.g., with trastuzumab) is mostly based on IHC. However, IHC-based approaches to determine the individual risk by testing a plurality of markers did not result in a reliable prognostic subclassification of bladder cancer that could help to resolve the diagnostic/therapeutic dilemma described above. Furthermore, testing by IHC generally suffers from a lack of sensitivity.

Therefore, there is clearly a need for a reliable, objective, quantitative and reproducible test system for the molecular subtyping of bladder cancer, which enables reliable individual risk assessment, facilitates the selection of suitable tumor treatment regimens (i.e., patient stratification), and allows prognosis and prediction of therapy success. Moreover, such test system should allow for decentralized testing that is suitable for a significant proportion of cancer patients.

These and other objects are solved by the present invention, which will be described in the following.

### SUMMARY OF THE INVENTION

The present invention is defined by the *in vitro* method of independent claim 1 and includes the method of claim 11. The present invention is also defined by the use of a kit of claim 15.

In one aspect, there is disclosed an *in vitro* method of identifying a molecular subtype of a tumor in a patient having bladder cancer, said method comprising determining the expression level of RNA transcript of at least one gene selected from the group consisting of human epidermal growth factor receptor 2 (HER2), estrogen receptor (ESR1), progesterone receptor (PGR), proliferation antigen Ki-67 (Ki67) and RacGTPase-activating protein 1 (RACGAP1) in a sample of the tumor.

In one example, the expression levels of RNA transcript of at least two, three or four genes selected from the group consisting of HER2, ESR1, PGR, Ki67 and RACGAP1 are determined.

In one example, the method comprises the steps:
(a) determining the expression level of RNA transcript of HER2 in a sample of the tumor; and
(b) determining the expression level of RNA transcript of ESR1 in a sample of the tumor,
wherein, optionally, the method further comprises determining the expression level of RNA transcript of at least one gene selected from Ki67 and RACGAP1 in a sample of the tumor.

In one example, there is disclosed an *in vitro* method of identifying a molecular subtype of a tumor in a patient having bladder cancer, said method comprising the steps:
(a) determining the expression level of RNA transcript of HER2 in a sample of the tumor;
(b) determining the expression level of RNA transcript of ESR1 in a sample of the tumor;
(c) determining the expression level of RNA transcript of PGR in a sample of the tumor; and, preferably,
(d) determining the expression level of RNA transcript of at least one gene selected from Ki67 and RACGAP1 in a sample of the tumor.

Specifically, the method of the invention comprises the steps:
(a) determining the expression level of RNA transcript of HER2 in a sample of the tumor;
(b) determining the expression level of RNA transcript of ESR1 in a sample of the tumor;
(c) determining the expression level of RNA transcript of PGR in a sample of the tumor; and
(d) determining the expression level of RNA transcript of Ki67 in a sample of the tumor.

In one example, determining the expression level of RNA transcript comprises determining whether the expression level of RNA transcript is lower or higher than a defined expression threshold of RNA transcript.

In one example, step (a) is performed before steps (b), (c) and (d).

In one example, step (d) is performed after steps (a), (b) and (c).

In one example, step (a) is performed before step (b), step (b) is performed before step (c), and step (c) is performed before step (d).

In one example, the molecular subtype is selected from the group comprising HER2-positive, triple-negative, luminal A and luminal B.

In one embodiment, an expression level of RNA transcript of HER2 which is higher than a defined expression threshold of RNA transcript of HER2 identifies the molecular subtype of the tumor as HER2-positive.

In one example, an expression level of RNA transcript of ESR1 which is higher than a defined expression threshold of RNA transcript of ESR1 indicates a positive prognosis, wherein, preferably, the positive prognosis comprises an increased probability of one or more of recurrence- free survival (RFS), progression-free survival (PFS), disease-specific survival (DSS), and distant recurrence-free survival, preferably RFS and/or PFS.

In one example, an expression level of RNA transcript of ESR1 which is lower than a defined expression threshold of RNA transcript of ESR1 indicates a negative prognosis, wherein, preferably, the negative prognosis comprises a reduced probability of one or more of recurrence- free survival (RFS), progression-free survival (PFS), disease-specific survival (DSS), and distant recurrence-free survival, preferably RFS and/or PFS.

In one example, an expression level of RNA transcript of HER2 which is lower than a defined expression threshold of RNA transcript of HER2;
- an expression level of RNA transcript of ESR1 which is lower than a defined expression threshold of RNA transcript of ESR1;
- an expression level of RNA transcript of PGR which is lower than a defined expression threshold of RNA transcript of PGR; and
- an expression level of RNA transcript of Ki67 which is lower or higher than a defined expression threshold of RNA transcript of Ki67
identify the molecular subtype of the tumor as triple-negative.

In one example, the molecular subtype is luminal A or luminal B.

In one example,
- an expression level of RNA transcript of HER2 which is lower than a defined expression threshold of RNA transcript of HER2;
- an expression level of RNA transcript of ESR1 which is higher than a defined expression threshold of RNA transcript of ESR1;
- an expression level of RNA transcript of PGR which is higher than a defined expression threshold of RNA transcript of PGR; and
- an expression level of RNA transcript of Ki67 which is higher than a defined expression threshold of RNA transcript of Ki67
identify the molecular subtype of the tumor as luminal B.

In one example,
- an expression level of RNA transcript of HER2 which is lower than a defined expression threshold of RNA transcript of HER2;
- an expression level of RNA transcript of ESR1 which is higher than a defined expression threshold of RNA transcript of ESR1;
- an expression level of RNA transcript of PGR which is higher than a defined expression threshold of RNA transcript of PGR; and
- an expression level of RNA transcript of Ki67 which is lower than a defined expression threshold of RNA transcript of Ki67
identify the molecular subtype of the tumor as luminal A.

In one example, an expression level of RNA transcript of HER2 which is lower than a defined expression threshold of RNA transcript of HER2;
- an expression level of RNA transcript of ESR1 which is higher than a defined expression threshold of RNA transcript of ESR1;
- an expression level of RNA transcript of PGR which is lower than a defined expression threshold of RNA transcript of PGR; and
- an expression level of RNA transcript of Ki67 which is lower or higher than a defined expression threshold of RNA transcript of Ki67
identify the molecular subtype of the tumor as luminal B.

In one example,
- an expression level of RNA transcript of HER2 which is lower than a defined expression threshold of RNA transcript of HER2;
- an expression level of RNA transcript of ESR1 which is lower than a defined expression threshold of RNA transcript of ESR1;
- an expression level of RNA transcript of PGR which is higher than a defined expression threshold of RNA transcript of PGR; and
- an expression level of RNA transcript of Ki67 which is higher than a defined expression threshold of RNA transcript of Ki67
identify the molecular subtype of the tumor as luminal B.

In one example,
- an expression level of RNA transcript of HER2 which is lower than a defined expression threshold of RNA transcript of HER2;
- an expression level of RNA transcript of ESR1 which is lower than a defined expression threshold of RNA transcript of ESR1;
- an expression level of RNA transcript of PGR which is higher than a defined expression threshold of RNA transcript of PGR; and
- an expression level of RNA transcript of K167 which is lower than a defined expression threshold of RNA transcript of Ki67
identify the molecular subtype of the tumor as luminal A.

In one example,
- the molecular subtype luminal A is associated with a probability of disease-specific survival (DSS) 10 years after treatment which is at least 10%, preferably at least 15%, higher than the probability of DSS 10 years after treatment associated with molecular subtype luminal B;
- the molecular subtype luminal A is associated with a probability of DSS 10 years after treatment which is at least 30%, preferably at least 35%, higher than the probability of DSS 10 years after treatment associated with molecular subtype HER2-positive or triple-negative; and/or
- the molecular subtype luminal B is associated with a probability of DSS 10 years after treatment which is at least 10%, preferably at least 15%, higher than the probability of DSS 10 years after treatment associated with molecular subtype HER2-positive or triple-negative.

In one example, the patient is a male patient, and
- the molecular subtype luminal A is associated with a probability of DSS 10 years after treatment which is at least 10%, preferably at least 15%, higher than the probability of DSS 10 years after treatment associated with molecular subtype luminal B;
- the molecular subtype luminal A is associated with a probability of DSS 10 years after treatment which is at least 15%, preferably at least 20%, higher than the probability of DSS 10 years after treatment associated with molecular subtype HER2-positive;
- the molecular subtype luminal A is associated with a probability of DSS 10 years after treatment which is at least 30%, preferably at least 35%, higher than the probability of DSS 10 years after treatment associated with molecular subtype triple-negative;
- the molecular subtype luminal B is associated with a probability of DSS 10 years after treatment which is at least 15%, preferably at least 20%, higher than the probability of DSS 10 years after treatment associated with molecular subtype triple-negative; and/or
- the molecular subtype HER2-positive is associated with a probability of DSS 10 years after treatment which is at least 10%, preferably at least 15%, higher than the probability of DSS 10 years after treatment associated with molecular subtype triple-negative.

In one example, the bladder cancer is non-muscle-invasive bladder cancer (NMIBC) or muscle-invasive bladder cancer (MIBC).

In one example, the sample is RNA extracted from the tumor.

In one example, the expression level of RNA transcript is determined by reverse transcription (RT) quantitative PCR (RT-qPCR).

In one example, the quantitative PCR is fluorescence-based quantitative real-time PCR.

In one example, the method comprises the use of ESR1-specific primers having a length of 15 to 30 nucleotides and comprising at least 10 contiguous nucleotides of the sequences of SEQ ID NOs: 1 and 2, and/or HER2-specific primers having a length of 15 to 30 nucleotides and comprising at least 10 contiguous nucleotides of the sequences of SEQ ID NOs: 4 and 5, and/or Ki67-specific primers having a length of 15 to 30 nucleotides and comprising at least 10 contiguous nucleotides of the sequences of SEQ ID NOs: 7 and 8, and/or PGR-specific primers having a length of 15 to 30 nucleotides and comprising at least 10 contiguous nucleotides of the sequences of SEQ ID NOs: 10 and 11, and/or RACGAP1-specific primers having a length of 15 to 30 nucleotides and comprising at least 10 contiguous nucleotides of the sequences of SEQ ID NOs: 13 and 14.

In one example, the method comprises the use of an ESR1-specific probe having a length of 20 to 35 nucleotides and comprising at least 15 contiguous nucleotides of the sequence of SEQ ID NO: 3, and/or a HER2-specific probe having a length of 20 to 35 nucleotides and comprising at least 15 contiguous nucleotides of the sequence of SEQ ID NO: 6, and/or a Ki67-specific probe having a length of 20 to 35 nucleotides and comprising at least 15 contiguous nucleotides of the sequence of SEQ ID NO: 9, and/or a PGR-specific probe having a length of 20 to 35 nucleotides and comprising at least 15 contiguous nucleotides of the sequence of SEQ ID NO: 12, and/or a RACGAP1-specific probe having a length of 20 to 35 nucleotides and comprising at least 15 contiguous nucleotides of the sequence of SEQ ID NO: 15.

In one example, the expression level is normalized against the (mean) expression level of one or more reference genes in the sample of the tumor.

In one example, the one or more reference genes are selected from the group comprising CALM2, B2M, RPL37A, GUSB, HPRT1 and GAPDH.

In a further aspect, there is disclosed a method of stratifying a bladder cancer patient for tumor treatment, said method comprising, as a first step, identifying a molecular subtype of a tumor in the bladder cancer patient using the *in vitro* method as defined above and, as a second step, selecting a tumor treatment regimen based on the molecular subtype identified by the *in vitro* method.

In one example, the molecular subtype is selected from the group comprising HER2-positive, triple-negative, luminal A and luminal B.

In one example, the bladder cancer is non-muscle-invasive bladder cancer (NMIBC) or muscle-invasive bladder cancer (MIBC).

In one example,
- the molecular subtype is luminal A, the bladder cancer is NMIBC, and the tumor treatment regimen comprises transurethral resection (TUR) and/or Bacillus Calmette-Guerin (BCG)-instillation, preferably TUR and BCG-instillation;
- the molecular subtype is luminal B, the bladder cancer is NMIBC, and the tumor treatment regimen comprises adjuvant chemotherapy with or without adjuvant endocrine therapy;
- the molecular subtype is HER2-positive, the bladder cancer is NMIBC, and the tumor treatment regimen comprises (neo)adjuvant chemotherapy with or without (neo)adjuvant anti- HER2 therapy;
- the molecular subtype is triple-negative, the bladder cancer is NMIBC, and the tumor treatment regimen comprises neoadjuvant chemotherapy;
- the molecular subtype is luminal A, the bladder cancer is MIBC, and the tumor treatment regimen comprises (i) cystectomy and (ii) adjuvant chemotherapy and/or adjuvant endocrine therapy, preferably adjuvant chemotherapy or adjuvant endocrine therapy;
- the molecular subtype is luminal B, the bladder cancer is MIBC, and the tumor treatment regimen comprises (i) cystectomy and (ii) adjuvant chemotherapy and/or adjuvant endocrine therapy, preferably adjuvant chemotherapy and adjuvant endocrine therapy;
- the molecular subtype is HER2-positive, the bladder cancer is MIBC, and the tumor treatment regimen comprises (i) cystectomy and (ii) adjuvant chemotherapy and/or adjuvant anti- HER2 therapy, preferably adjuvant chemotherapy and adjuvant anti-HER2 therapy; and/or
- the molecular subtype is triple-negative, the bladder cancer is MIBC, and the tumor treatment regimen comprises neoadjuvant chemotherapy with or without subsequent cystectomy.

In another aspect, there is disclosed a use in a method of treatment of bladder cancer, the method comprising, as a first step, stratifying a bladder cancer patient for tumor treatment using the method as defined above and, as a second step, providing the selected tumor treatment regimen to the bladder cancer patient.

In yet another aspect, there is disclosed the use of a kit for identifying a molecular subtype of a tumor in a bladder cancer patient by means of reverse transcription (RT) quantitative PCR (RT-qPCR), said kit comprising at least one pair of primers and at least one probe that are specific for a gene selected from the group consisting of HER2, ESR1, PGR, Ki67 and RACGAP1.

In one example, the kit comprises specific pairs of primers and specific probes for at least two, three or four genes selected from the group consisting of HER2, ESR1, PGR, Ki67 and RACGAP1.

In one example, the kit comprises:
- at least one pair of HER2-specific primers and at least one HER2-specific probe;
- at least one pair of ESR1-specific primers and at least one ESR1-specific probe; and, optionally,
- at least one pair of Ki67-specific primers and at least one Ki67-specific probe; and/or
- at least one pair of RACGAP 1-specific primers and at least one RACGAP1-specific probe.

In one example, the kit further comprises:
- at least one pair of PGR-specific primers and at least one PGR-specific probe.

In one example, the invention relates to the use of a kit for identifying a molecular subtype of a tumor in a bladder cancer patient by means of reverse transcription (RT) quantitative PCR (RT-qPCR), said kit comprising:
- at least one pair of HER2-specific primers and at least one HER2-specific probe;
- at least one pair of ESR1-specific primers and at least one ESR1-specific probe;
- at least one pair of PGR-specific primers and at least one PGR-specific probe; and
- at least one pair of Ki67-specific primers and at least one Ki67-specific probe; and/or
- at least one pair of RACGAP1-specific primers and at least one RACGAP1-specific probe.

In one example, the kit comprises:
- at least one pair of HER2-specific primers and at least one HER2-specific probe;
- at least one pair of ESR1-specific primers and at least one ESR1-specific probe;
- at least one pair of PGR-specific primers and at least one PGR-specific probe; and
- at least one pair of Ki67-specific primers and at least one Ki67-specific probe.

In one example, the quantitative PCR is fluorescence-based quantitative real-time PCR.

In one example, detection of the probe is based on amplification-mediated probe displacement.

In one example, the probe is a dual-label probe comprising a fluorescence reporter moiety and a fluorescence quencher moiety.

In one example, the kit further comprises a reverse transcriptase and a DNA polymerase.

In one example, the reverse transcriptase and the DNA polymerase are provided in the form of an enzyme-mix which allows a one-step reverse transcription (RT) quantitative PCR (RT- qPCR).

In one example, the kit further comprises at least one pair of reference gene-specific primers and at least one reference gene-specific probe.

In one example, the reference gene is one or more selected from the group comprising CALM2, B2M, RPL37A, GUSB, HPRT1 and GAPDH.

In one example, the kit further comprises at least one control RNA sample. In one embodiment, the primers provide an amplicon size of less than 120 bp.

In one example, the ESR1-specific primers have a length of 15 to 30 nucleotides and comprise at least 10 contiguous nucleotides of the sequences of SEQ ID NOs: 1 and 2, and/or the HER2- specific primers have a length of 15 to 30 nucleotides and comprise at least 10 contiguous nucleotides of the sequences of SEQ ID NOs: 4 and 5, and/or the Ki67-specific primers have a length of 15 to 30 nucleotides and comprise at least 10 contiguous nucleotides of the sequences of SEQ ID NOs: 7 and 8, and/or the PGR-specific primers have a length of 15 to 30 nucleotides and comprise at least 10 contiguous nucleotides of the sequences of SEQ ID NOs: 10 and 11, and/or the RACGAP1-specific primers have a length of 15 to 30 nucleotides and comprise at least 10 contiguous nucleotides of the sequences of SEQ ID NOs: 13 and 14.

In one example, the ESR1-specific probe has a length of 20 to 35 nucleotides and comprises at least 15 contiguous nucleotides of the sequence of SEQ ID NO: 3, and/or the HER2-specific probe has a length of 20 to 35 nucleotides and comprises at least 15 contiguous nucleotides of the sequence of SEQ ID NO: 6, and/or the Ki67-specific probe has a length of 20 to 35 nucleotides and comprises at least 15 contiguous nucleotides of the sequence of SEQ ID NO: 9, and/or the PGR-specific probe has a length of 20 to 35 nucleotides and comprises at least 15 contiguous nucleotides of the sequence of SEQ ID NO: 12, and/or the RACGAP1-specific probe has a length of 20 to 35 nucleotides and comprises at least 15 contiguous nucleotides of the sequence of SEQ ID NO: 15.

In one example, the bladder cancer is non-muscle-invasive bladder cancer (NMIBC) or muscle-invasive bladder cancer (MIBC).

In a further aspect, the disclosure relates to the use of the expression level of RNA transcript of ESR1 and/or the expression level of RNA transcript of PGR as prognostic biomarker(s) for bladder cancer.

In one example, the expression level of RNA transcript of ESR1 or the expression level of RNA transcript of PGR is used as a singular prognostic biomarker.

In one example, the bladder cancer is non-muscle-invasive bladder cancer (NMIBC) or muscle-invasive bladder cancer (MIBC).

In one example,
- an expression level of RNA transcript of ESR1 which is higher than a defined expression threshold of RNA transcript of ESR1 indicates a positive prognosis; and/or
- an expression level of RNA transcript of PGR. which is higher than a defined expression threshold of RNA transcript of PGR indicates a positive prognosis.

In one example, the positive prognosis comprises an increased probability of one or more of prolonged disease-specific survival (DSS), recurrence-free survival (RFS), progression-free survival (PFS) and distant recurrence-free survival, preferably DSS.

In one example,
- an expression level of RNA transcript of ESR1 which is lower than a defined expression threshold of RNA transcript of ESR1 indicates a negative prognosis; and/or
- an expression level of RNA transcript of PGR which is lower than a defined expression threshold of RNA transcript of PGR indicates a negative prognosis.

In one example, the negative prognosis comprises a reduced probability of one or more of prolonged disease-specific survival (DSS), recurrence-free survival (RFS), progression-free survival (PFS) and distant recurrence-free survival, preferably DSS.

In one example, the bladder cancer is of the HER2-positive molecular subtype and an expression level of RNA transcript of ESR1 which is higher than a defined expression threshold of RNA transcript of ESR1 indicates a positive prognosis, wherein, preferably, the positive prognosis comprises an increased probability of one or more of recurrence-free survival (RFS), progression-free survival (PFS), disease-specific survival (DSS), and distant recurrence-free survival, preferably RFS and/or PFS.

In one example, the bladder cancer is of the HER2-positive molecular subtype and an expression level of RNA transcript of ESR1 which is lower than a defined expression threshold of RNA transcript of ESR1 indicates a negative prognosis, wherein, preferably, the negative prognosis comprises a reduced probability of one or more of recurrence-free survival (RFS), progression-free survival (PFS), disease-specific survival (DSS), and distant recurrence-free survival, preferably RFS and/or PFS.

### BRIEF DESCRIPTION OF THE FIGURES

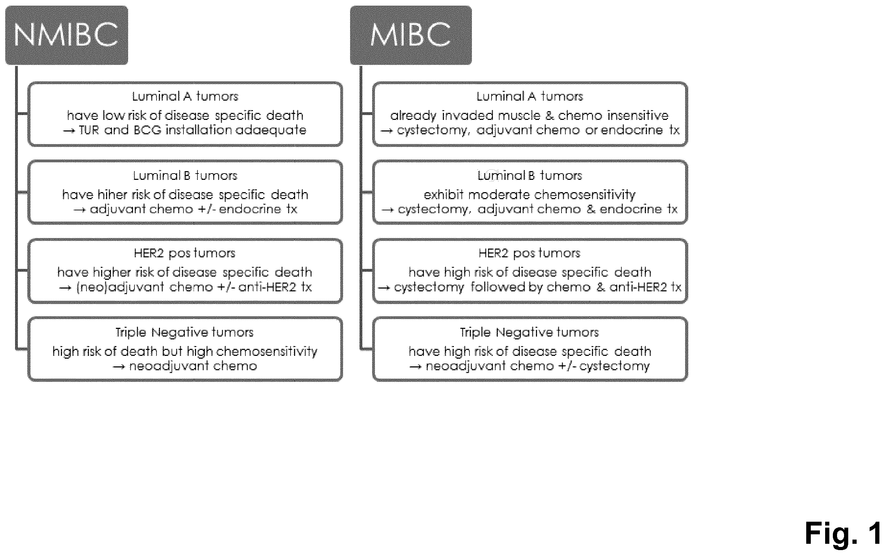

**Figure 1** outlines tumor treatment regimens (tx = therapy) for non-muscle-invasive bladder cancer (NMIBC) or muscle-invasive bladder cancer (MIBC) of the molecular subtypes luminal A, luminal B, HER2-positive and triple-negative in accordance with the present invention.
**Figure 2** summarizes the classification scheme based on combinatorial usage of dichotomized single marker detection.
**Figure 3** depicts the cut-off values for the individual markers to be used for bladder cancer subtype classification according to the scheme of **Figure 2****,** when using the BladderTyper RT-qPCR kit as described herein on a Siemens Versant kPCR system.
**Figure 4** depicts the data distribution of the normalized candidate gene expression of ESR1, PGR, HER2 and Ki67 mRNA when performing the BladderTyper RT-qPCR kit as described herein in 82 non-muscle-invasive bladder cancer specimens.
**Figure 5** depicts a partitioning test to evaluate the prognostic value of subtyping based on the mRNA expression level of HER2, ESR1 and PGR for the 5-years disease-specific survival (DSS) rate of bladder cancer patients. Available data from 82 tumors were stratified by the classification scheme depicted in **Figure 2** using the cut-off values for the individual genes depicted in **Figure 3****.**

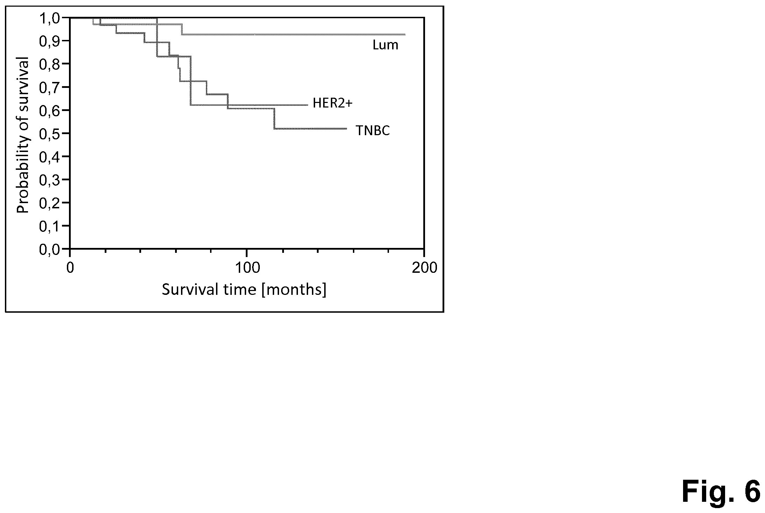

**Figure 6** depicts a Kaplan Meier analysis of disease-specific survival of bladder cancer patients with HER2-positive (HER2+), luminal A/B (Lum) or triple-negative (TNBC) tumors, wherein the molecular subtype of the tumor was identified in accordance with the present invention, based on the mRNA expression levels of HER2, ESR1 and PGR. The luminal A/B subtypes (Lum), as defined by the present inventors, are associated with a superior disease-specific survival rate after 5 years, which differs significantly from that associated with HER2-positive bladder tumors or triple-negative bladder cancer (90% vs 80% vs 70%, respectively; p=0,02).
**Figure 7** depicts a partitioning test to evaluate the prognostic value of subtyping based on the mRNA expression level of HER2, ESR1, PGR and Ki67 for the 5-years disease-specific survival (DSS) rate of bladder cancer patients. Available data from 82 tumors were stratified by the classification scheme depicted in **Figure 2** using the cut-off values for the individual genes depicted in **Figure 3****.** For luminal tumors, a cut-off value for Ki67 of 36,12 differentiated luminal A from luminal B tumors the best.

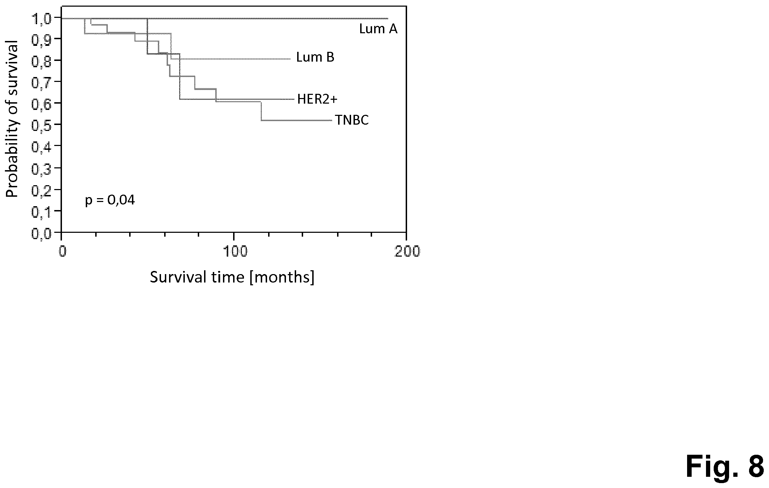

**Figure 8** depicts a Kaplan Meier analysis of disease-specific survival (DSS; cancer-specific death after transurethral resection in months) of bladder cancer patients with luminal A (LumA), luminal B (LumB), HER2-positive (HER2+) or triple-negative bladder cancer (TNBC), wherein the molecular subtype of the tumor was identified in accordance with the present invention, based on the mRNA expression levels of HER2, ESR1, PGR and Ki67. The luminal A subtype, as defined by the present inventors, has a significantly better DSS rate after 8 years than luminal B, HER-positive and triple-negative tumors (p=0,04; 100% vs. 80% for luminal B vs. 70% for HER2-positive vs. 60% for triple-negative bladder cancer).
**Figure 9** depicts a partitioning test to evaluate the prognostic value of subtyping based on the mRNA expression level of HER2, ESR1, PGR and Ki67 for the 5-years disease-specific survival (DSS) rate of male bladder cancer patients. Available data from 67 tumors were stratified by the classification scheme depicted in **Figure 2** using the cut-off values for the individual genes depicted in **Figure 3****.** For luminal tumors, a cut-off value for Ki67 of 36,12 differentiated luminal A from luminal B tumors the best.

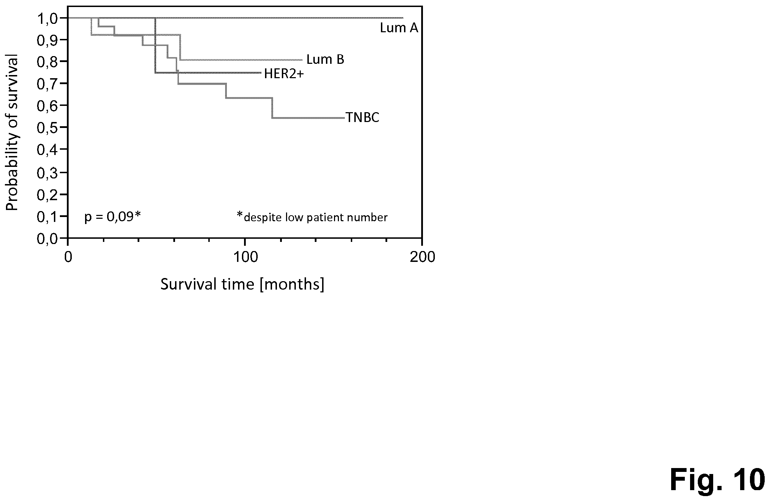

**Figure 10** depicts a Kaplan Meier analysis of disease-specific survival (DSS; cancer-specific death after transurethral resection in months) of male bladder cancer patients with luminal A (LumA), luminal B (LumB), HER2-positive (HER2+) or triple-negative bladder cancer (TNBC), wherein the molecular subtype of the tumor was identified in accordance with the present invention, based on the mRNA expression levels of HER2, ESR1, PGR and Ki67. The luminal A subtype, as defined by the present inventors, has a significantly better DSS rate after 8 years than luminal B, HER-positive and triple-negative tumors (p=0,09; 100% vs. 80% for luminal B vs. 75% for HER2-positive vs. 60% for triple-negative bladder cancer).
**Figure 11** depicts a Kaplan Meier analysis of disease-specific survival (DSS; cancer-specific death after transurethral resection in months) of ESR1-positive bladder cancer (ESR1+) versus ESR1-negative bladder cancer (ESR1-), wherein the ESR1-positivity of the tumor was identified in accordance with the present invention, based on the mRNA expression level of ESR1. ESR1-positive tumors have a significantly better disease-specific survival rate (p=0,02).
**Figure 12** depicts a Kaplan Meier analysis of disease-specific survival (DSS; cancer-specific death after transurethral resection in months) of PGR-positive bladder cancer (PGR+) versus PGR-negative bladder cancer (PGR-), wherein the PGR-positivity of the tumor was identified in accordance with the present invention, based on the mRNA expression level of PGR. PGR-positive tumors have a significantly better disease-specific survival rate (p=0,01).
**Figure 13** depicts a Kaplan Meier analysis of disease-specific survival (DSS; in months) of the following MIBC "subtypes": HER2+, HER2-/ESR1+, HER2-/ESR1-/Ki67+ and HER2-/ESR1-/Ki67- (p<0,0001).
**Figure 14** depicts a partitioning test to evaluate the prognostic value of subtyping based on the mRNA expression level of HER2, ESR1 and Ki67 for the disease-specific survival (DSS) rate of MIBC patients.
**Figure 15** depicts a partitioning test to evaluate the prognostic value of subtyping based on the mRNA expression level of HER2 and ESR1 for the progression-free survival (PFS) rate of NMIBC patients.
**Figure 16** depicts a Kaplan Meier analysis of recurrence-free survival (RFS; in months) of the following NMIBC "subtypes": HER2-, HER2+/ESR1- and HER2+/ESR1+.
**Figure 17** depicts a Kaplan Meier analysis of progression-free survival (PFS; in months) of the following NMIBC "subtypes": HER2-, HER2+/ESR1- and HER2+/ESR1+.
**Figure 18** depicts a proportional hazards model of a multivariate analysis of progression-free survival (PFS; in months) depending on HER2 and ESR1 mRNA expression status in NMIBC tumors.
**Figure 19** shows the subtype distribution across a total T1 NMIBC cohort.
**Figure 20** depicts a Kaplan Meier analysis of recurrence-free survival (RFS; in months) based on predefined subtypes in an NMIBC validation cohort.
**Figure 21** depicts a multivariate analysis of the prognostic value of predefined molecular subtyping for RFS in an NMIBC validation cohort after adjustment for CIS, tumor size, gender and central grading according to the WHO 2004 scheme.
**Figure 22** depicts a Kaplan Meier analysis of progression-free survival (PFS; in months) based on predefined subtypes in an NMIBC validation cohort.
**Figure 23** depicts a multivariate analysis of the prognostic value of predefined molecular subtyping for PFS in an NMIBC validation cohort after adjustment for CIS, tumor size, gender and central grading according to the WHO 2004 scheme.
**Figure 24** depicts a Kaplan Meier analysis of recurrence-free survival (RFS; in months) of NMIBC patients stratified by intermediate ERBB2 mRNA levels at 40.1.
**Figure 25** depicts a Kaplan Meier analysis of progression-free survival (PFS; in months) of NMIBC patients stratified by intermediate ERBB2 mRNA levels at 40.1.
**Figure 26** depicts a Kaplan Meier analysis of recurrence-free survival (RFS; in months) of male NMIBC patients stratified by intermediate ERBB2 mRNA levels at 40.1.
**Figure 27** depicts a Kaplan Meier analysis of progression-free survival (PFS; in months) of male NMIBC patients stratified by intermediate ERBB2 mRNA levels at 40.1.
**Figure 28** depicts a Kaplan Meier analysis of recurrence-free survival (RFS; in months) in NMIBC patients stratified by intermediate ERBB2 mRNA levels at 40.1 and grade (WHO Grade 1973).
**Figure 29** depicts a Kaplan Meier analysis of progression-free survival (PFS; in months) of NMIBC patients stratified by ERBB2 mRNA levels at 40.1 +/- instillation therapy.
**Figure 30** depicts a Kaplan Meier analysis of progression-free survival (PFS; in months) of NMIBC patients stratified by intermediate ERBB2 mRNA levels at 40.1 +/- instillation therapy.
**Figure 31** depicts a Scatter Plot analysis of ERBB2 (HER2), ESR1, PGR or MKI67 (Ki67) mRNA levels in centrally assessed grade 1, 2 and 3 bladder cancer tumors.
**Figure 32** depicts a Scatter Plot analysis of ERBB2 (HER2), ESR1, PGR or MKI67 (Ki67) mRNA levels of stage Ta (pTa) and stage T2 (pT1) bladder cancer tumors.

Other objects, advantages and features of the present invention will become apparent from the following detailed description, in particular when considered in conjunction with the accompanying figures.

### DETAILED DESCRIPTION OF THE INVENTION

Although the present invention is described in detail below, it is to be understood that this invention is not limited to the particular methodologies, protocols and reagents described herein as these may vary. It is also to be understood that the terminology used herein is for the purpose of describing particular embodiments only, and is not intended to limit the scope of the present invention, which will be limited only by the appended claims. Unless defined otherwise, all technical and scientific terms used herein have the same meanings as commonly understood by one of ordinary skill in the art.

In the following, certain elements of the present invention will be described. These elements may be listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments, which combine the explicitly described embodiments with any number of the disclosed and/or preferred elements. Furthermore, any permutations and combinations of all described elements in this application should be considered disclosed by the description of the present application unless the context indicates otherwise. For example, as will be clear to a person skilled in the art, the specific embodiments disclosed herein relating to the expression levels of RNA transcript of particular genes (being higher or lower than defined expression thresholds of RNA transcript of the particular genes) and the molecular subtypes based thereon can be combined so as to allow for the identification of a molecular subtype of a given tumor.

Preferably, the terms used herein are defined as described in "A multilingual glossary of biotechnological terms (IUPAC Recommendations)", H.G.W. Leuenberger, B. Nagel, and H. Kölbl, Eds., Helvetica Chimica Acta, CH-4010 Basel, Switzerland, (1995).

The practice of the present invention will employ, unless otherwise indicated, conventional methods of chemistry, biochemistry, cell biology, immunology, and recombinant DNA techniques which are explained in the literature in the field (cf., e.g., Molecular Cloning: A Laboratory Manual, 3rd Edition, J. Sambrook et al. eds., Cold Spring Harbor Laboratory Press, Cold Spring Harbor 2000) .

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps although in some embodiments such other member, integer or step or group of members, integers or steps may be excluded, i.e. the subject-matter consists in the inclusion of a stated member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

In one aspect, the disclosure relates to an *in vitro* method of identifying a molecular subtype of a tumor in a patient having bladder cancer, said method comprising determining the expression level of RNA transcript of at least one gene selected from the group consisting of human epidermal growth factor receptor 2 (HER2), estrogen receptor (ESR1), progesterone receptor (PGR), proliferation antigen Ki-67 (Ki67) and RacGTPase-activating protein 1 (RACGAP1) in a sample of the tumor.

In one embodiment, the expression levels of RNA transcript of at least two, three or four genes selected from the group consisting of HER2, ESR1, PGR, Ki67 and RACGAP1 are determined.

In one embodiment, the method comprises the steps:
(a) determining the expression level of RNA transcript of HER2 in a sample of the tumor; and
(b) determining the expression level of RNA transcript of ESR1 in a sample of the tumor,
wherein, optionally, the method further comprises determining the expression level of RNA transcript of at least one gene selected from Ki67 and RACGAP 1 in a sample of the tumor, wherein Ki67 is particularly preferred.

In one embodiment, the invention relates to an *in vitro* method of identifying a molecular subtype of a tumor in a patient having bladder cancer, said method comprising the steps:
(a) determining the expression level of RNA transcript of HER2 in a sample of the tumor;
(b) determining the expression level of RNA transcript of ESR1 in a sample of the tumor; and
(c) determining the expression level of RNA transcript of PGR in a sample of the tumor.

In a preferred embodiment, the method further comprises the step:
(d) determining the expression level of RNA transcript of at least one gene selected from Ki67 and RACGAP1 in a sample of the tumor, wherein Ki67 is particularly perferred.

In one embodiment, the method comprises the steps:
(a) determining the expression level of RNA transcript of HER2 in a sample of the tumor;
(b) determining the expression level of RNA transcript of ESR1 in a sample of the tumor;
(c) determining the expression level of RNA transcript of PGR in a sample of the tumor; and
(d) determining the expression level of RNA transcript of Ki67 in a sample of the tumor.

In one embodiment, said method comprises the determination of the expression level, in particular the expression level of RNA transcript, of one or more additional non-reference genes.

In one embodiment, said method does not comprise the determination of the expression level, in particular the expression level of RNA transcript, of more than three, two or one additional non-reference gene.

In one embodiment, said method does not comprise the determination of the expression level, in particular the expression level of RNA transcript, of any additional non-reference gene. In other words, no expression level, in particular no expression level of RNA transcript, of a gene other than HER2, ESR1, PGR and, optionally, at least one gene selected from Ki67 and RACGAP1, and, optionally, one or more reference genes is determined. In one embodiment, no expression level, in particular no expression level of RNA transcript, of a gene other than HER2, ESR1, PGR, Ki67 and, optionally, one or more reference genes is determined. In another embodiment, no expression level, in particular no expression level of RNA transcript, of a gene other than HER2, ESR1 and, optionally, at least one gene selected from Ki67 and RACGAP1, preferably Ki67, and, optionally, one or more reference genes is determined. In yet another embodiment, no expression level, in particular no expression level of RNA transcript, of a gene other than HER2, ESR1 and, optionally, one or more reference genes is determined.

In one embodiment, the expression levels of RNA transcript of a maximum of 7, preferably 6, more preferably 5, even more preferably 4 different non-reference genes are determined.

In one embodiment, said method does not comprise any other diagnostic steps, such as histological grading or determining the lymph nodal status. In one embodiment, said method does not comprise any steps involving immunohistochemistry (IHC).

The term "tumor", as used herein, refers to all neoplastic cell growth and proliferation whether malignant or benign, and all pre-cancerous and cancerous cells and tissues. In one embodiment of the present invention, the tumor is a solid tumor. In one embodiment, the tumor is a bladder or urethral tumor or is derived from a bladder or urethral tumor (e.g., by metastasis). The term "bladder", as used herein, refers to the urinary bladder. As used herein, the term "cancer" includes a disease characterized by aberrantly regulated cellular growth, proliferation, differentiation, adhesion, and/or migration. The term cancer according to the invention also comprises cancer metastases. The terms "tumor" and "cancer" may be used interchangeably herein.

The term "metastasis" is meant to refer to the spread of cancer cells from their original site to another part of the body. The formation of metastasis is a very complex process and depends on detachment of malignant cells from the primary tumor, invasion of the extracellular matrix, penetration of the endothelial basement membranes to enter the body cavity and vessels, and then, after being transported by the blood, infiltration of target organs. Finally, the growth of a new tumor at the target site depends on angiogenesis. Tumor metastasis often occurs even after the removal of the primary tumor because tumor cells or components may remain and develop metastatic potential.

The term "bladder cancer" relates to a type of cancer originating from bladder or urethral tissue. In one embodiment, the bladder cancer is non-muscle-invasive bladder cancer (NMIBC) or muscle-invasive bladder cancer (MIBC). Occasionally, bladder cancer is metastatic. Common sites of metastasis include bone, liver, lung and brain. Bladder cancer occurs in humans and other mammals. While the majority of human cases occur in men, female bladder cancer can also occur. Generally, treatment of bladder cancer may include surgery, medications (such as immunotherapy and/or chemotherapy and/or immunotherapy by BCG), radiation and/or targeted therapy.

Nearly all bladder cancers start in the urothelium. As the cancer grows into or through the other layers in the bladder, it then becomes more advanced, wherein the classification into stages follows the progression of infiltration of the tumor in deeper tissue layers. The stages are defined as: Ta (pTa): Non-invasive papillary carcinoma, Tis (pTis): Non-invasive flat carcinoma (flat carcinoma in situ, or CIS), T1 (pT1): The tumor has grown from the layer of cells lining the bladder into the connective tissue below but is still considered being a NMIBC, T2 (pT2): The tumor has grown into the muscle layer (MIBC), T3 (pT3): The tumor has grown through the muscle layer of the bladder and into the fatty tissue layer that surrounds it; T4 (pT4): The tumor has spread beyond the fatty tissue and into nearby organs or structures. It may be growing into any of the following: the stroma (main tissue) of the prostate, the seminal vesicles, uterus, vagina, pelvic wall, or abdominal wall.

The term "patient", as used herein, refers to any organism such as vertebrate, particularly any mammal, including both a human and another mammal, e.g., an animal such as a rodent, a rabbit, or a monkey. The rodent may be a mouse, rat, hamster, guinea pig, or chinchilla. Preferably, the patient is a human.

According to the present invention, the term "RNA transcript" includes and preferably relates to "mRNA" which means "messenger RNA" and relates to a "transcript" which encodes a peptide or protein. mRNA typically comprises a 5' non translated region (5'-UTR), a protein or peptide coding region and a 3' non translated region (3'-UTR). mRNA has a limited halftime in cells and *in vitro.*

The gene HER2 (also referred to as ERBB2; location: 17q12, annotation: chromosome: 17; NC_000017.10; UniProt: P04626) encodes a member of the epidermal growth factor (EGF) receptor family of receptor tyrosine kinases. Amplification and/or overexpression of this gene have been reported in numerous cancers, including breast and ovarian tumors. In the NCBI database, two mRNA variants for HER2 are listed which code for two protein versions. Protein and mRNA sequences can be found under the accession numbers NM_001005862.1 (receptor tyrosine-protein kinase erbB-2 isoform b) and NM_004448.2 (receptor tyrosine-protein kinase erbB-2 isoform a precursor).

The gene ESR1 (location: 6q25, annotation: chromosome 6, NC_000006.11; UniProt: P03372) encodes an estrogen receptor (ER), a ligand-activated transcription factor composed of several domains important for hormone binding, DNA binding, and activation of transcription. Estrogen receptors are known to be involved in pathological processes including breast cancer, endometrial cancer, and osteoporosis. Four ESR1 mRNA variants are known, wherein the transcript variants differ in the 5' UTR and/or use different promoters, but each variant codes for the same protein.

The gene PGR (also referred to as PR; location: 11q22-q23, annotation: chromosome: 11; NC_000011.9; UniProt: P06401) encodes the progesterone receptor. Steroid hormones such as progesterone and their receptors are involved in the regulation of eukaryotic gene expression and affect cellular proliferation and differentiation in target tissues. This gene uses two distinct promoters and translation start sites in the first exon to produce two mRNA isoforms, A and B. The two isoforms are identical except for the additional 165 amino acids found in the N-terminus of isoform B.

The gene Ki-67 or MKI67 (Ki67; location: 10q26.2, annotation: chromosome: 10; NC_000010.10; UniProt: P46013) encodes a nuclear protein that is associated with and may be necessary for cellular proliferation. Two mRNA variants have been described. A related pseudogene exists on chromosome 10.

The gene RACGAP1 (location: 12q13.12, annotation: chromosome: 12; NC_000012.11; UniProt: Q9H0H5) encodes for RacGTPase-activating protein 1. Three splice variants have been described, all encoding for the same protein. RACGAP1 is a component of the central spindlin complex and plays key roles in controlling growth-related processes and differentiation.

The term "expression level", as used herein, refers to the expression of a particular gene (e.g., HER2, ESR1, PGR or Ki67) so as to produce transcript and/or protein. According to the present invention, the expression level is determined on the RNA transcript level, in particular mRNA level (transcriptional level), for example, by measuring the transcribed mRNA (e.g., via northern blot), by reverse transcription (RT) quantitative PCR (RT-qPCR) or by directly staining the mRNA (e.g., via *in situ* hybridization).

In one embodiment, the term "sample of the tumor" refers to a tumor tissue sample isolated from the cancer patient (e.g., a biopsy or resection tissue of the tumor). In a preferred embodiment, the tumor tissue sample is a cryo-section of a tumor tissue sample or is a chemically fixed tumor tissue sample. In a more preferred embodiment, the tumor tissue sample is a formalin-fixed and paraffin-embedded (FFPE) tumor tissue sample. In one embodiment, the sample of the tumor is (total) RNA extracted from the tumor tissue sample. In a particularly preferred embodiment, the sample of the tumor is (total) RNA extracted from a FFPE tumor tissue sample. Those skilled in the art are able to perform RNA extraction procedures. For example, total RNA from a 5 to 10 µm curl of FFPE tumor tissue can be extracted using the High Pure RNA Paraffin Kit (Roche, Basel, Switzerland) or, the XTRAKT RNA Extraction Kit XL (Stratifyer Molecular Pathology, Cologne, Germany) or RNXtract^{®} Extraction Kit (BioNTech Diagnostics GmbH, Mainz, Germany). It is also possible to store the sample material to be used/tested in a freezer and to carry out the method of the present invention at an appropriate point in time after thawing the respective sample material. The sample may be obtained from the cancer patient prior to initiation of a therapeutic treatment, during the therapeutic treatment and/or after the therapeutic treatment, i.e. prior to, during or following the administration of cancer therapy.

The term "molecular subtype of a tumor" (or "molecular subtype of a cancer"), as used herein, refers to subtypes of a tumor/cancer that are characterized by distinct molecular profiles, e.g., gene expression profiles.

In one embodiment, the molecular subtype is selected from the group comprising, preferably consisting of, HER2-positive, triple-negative (also referred to as "basal-like"), luminal A and luminal B. The term "basal-like" refers to the fact that such tumors have some similarity in gene expression to that of basal epithelial cells. The term "luminal" derives from the similarity in gene expression between the tumors and the luminal epithelium.

In one embodiment, the expression levels of RNA transcript of HER2, ESR1 and Ki67 are determined, and the molecular subtype is selected from the group comprising, preferably consisting of, HER2+, HER2-/ESR1+, HER2-/ESR1-/Ki67+ and HER2-/ESR1-/Ki67-. In one embodiment, said molecular subtype relates to MIBC.

The molecular subtypes differ markedly in clinical outcome and response to therapy. The term "clinical outcome" is defined as the clinical result of a disease, in particular following a treatment, e.g., reduction or amelioration of symptoms. In one embodiment, poor clinical outcome comprises a relative reduction in or more of disease-specific survival (DSS), recurrence-free survival (RFS), progression-free survival (PFS) and distant recurrence-free survival. The term "recurrence" with respect to cancer includes re-occurrence of tumor cells at the same site and organ of the origin disease, metastasis that can appear even many years after the initial diagnosis and therapy of cancer, or local events such as infiltration of tumor cells into regional lymph nodes. "Distant recurrence" refers to a scenario, where the cancer cells have spread (metastasized) to a distant part (i.e., another organ) of the body beyond the regional lymph nodes. Recurrence-free survival is generally defined as the time from randomization to the first of recurrence, relapse, second cancer, or death. Progression-free survival is the time that passes from a certain date (generally the first day of treatment, or the day in which a patient is enrolled in a clinical trial) and the date on which disease "progresses" or the date on which the patient dies, from any cause. The terms "DSS" and "CSS" (for "cancer-specific survival") may be used interchangeably herein.

In one embodiment, the molecular subtypes luminal A and B are associated with a probability of disease-specific survival 5 years after treatment which is at least 10%, preferably at least 15% higher than the probability of disease-specific survival 5 years after treatment associated with molecular subtype triple-negative or HER2-positive. In one embodiment, the molecular subtypes luminal A and B, preferably luminal A, are associated with a positive prognosis for the patient. In one embodiment, the positive prognosis comprises an increased probability of one or more of prolonged disease-specific survival (DSS), recurrence-free survival (RFS), progression-free survival (PFS) and distant recurrence-free survival, preferably DSS.

In one embodiment,
- the molecular subtype luminal A is associated with a probability of disease-specific survival (DSS) 10 years after treatment which is at least 10%, preferably at least 15%, higher than the probability of DSS 10 years after treatment associated with molecular subtype luminal B;
- the molecular subtype luminal A is associated with a probability of DSS 10 years after treatment which is at least 30%, preferably at least 35%, higher than the probability of DSS 10 years after treatment associated with molecular subtype HER2-positive or triple-negative; and/or
- the molecular subtype luminal B is associated with a probability of DSS 10 years after treatment which is at least 10%, preferably at least 15%, higher than the probability of DSS 10 years after treatment associated with molecular subtype HER2-positive or triple-negative.

In one embodiment, the patient is a male patient, and
- the molecular subtype luminal A is associated with a probability of DSS 10 years after treatment which is at least 10%, preferably at least 15%, higher than the probability of DSS 10 years after treatment associated with molecular subtype luminal B;
- the molecular subtype luminal A is associated with a probability of DSS 10 years after treatment which is at least 15%, preferably at least 20%, higher than the probability of DSS 10 years after treatment associated with molecular subtype HER2-positive;
- the molecular subtype luminal A is associated with a probability of DSS 10 years after treatment which is at least 30%, preferably at least 35%, higher than the probability of DSS 10 years after treatment associated with molecular subtype triple-negative;
- the molecular subtype luminal B is associated with a probability of DSS 10 years after treatment which is at least 15%, preferably at least 20%, higher than the probability of DSS 10 years after treatment associated with molecular subtype triple-negative; and/or
- the molecular subtype HER2-positive is associated with a probability of DSS 10 years after treatment which is at least 10%, preferably at least 15%, higher than the probability of DSS 10 years after treatment associated with molecular subtype triple-negative.

The term "(therapeutic) treatment", in particular in connection with the treatment of cancer, as used herein, relates to any treatment which improves the health status and/or prolongs (increases) the lifespan of a patient. Said treatment may eliminate cancer, reduce the size or the number of tumors in a patient, arrest or slow the development of cancer in a patient, inhibit or slow the development of new cancer in a patient, decrease the frequency or severity of symptoms in a patient, and/or decrease recurrences in a patient who currently has or who previously has had cancer. In one embodiment, the terms "treatment" and "therapeutic treatment" are meant to refer to one or more of surgical removal of the primary tumor, chemotherapy, anti-hormonal therapy, radiation therapy and immunotherapy/targeted therapy.

Adjuvant therapy is a treatment that is given in addition to the primary, main or initial treatment. The surgeries and complex treatment regimens used in cancer therapy have led the term to be used mainly to describe adjuvant cancer treatments. An example of adjuvant therapy is the additional treatment (e.g., chemotherapy) usually given after surgery (post-surgically), where all detectable disease has been removed, but where there remains a statistical risk of relapse due to occult disease. Neoadjuvant therapy is treatment given before the primary, main or initial treatment (e.g., pre-surgical chemotherapy).

In accordance with the present invention, the step of "determining the expression level of RNA transcript" may comprise (i) measuring the expression level of RNA transcript and (ii) analyzing the measured expression level of RNA transcript (e.g., by comparison to a reference expression level, such as a defined expression threshold), wherein the order of measuring the expression level of RNA transcript may or may not be independent of the order of analyzing the measured expression level of RNA transcript.

In one embodiment, determining the expression level of RNA transcript comprises determining whether the expression level of RNA transcript is lower or higher than a defined expression threshold of RNA transcript (= dichotomization). In cases where the expression level is equal to the defined expression threshold, the expression level is considered to belong to the group of expression levels that are higher than the defined expression threshold. Thus, the wording "higher than a defined expression threshold", as used herein, includes expression levels that are higher than or equal to the defined expression threshold. Expression levels that are "higher than a defined expression threshold" may also be referred to as "expression-positive", whereas expression levels that are "lower than a defined expression threshold" may also be referred to as "expression-negative".

The term "defined expression threshold of RNA transcript", as used herein, may refer to the mean cut-off value (in short: cut-off) calculated from a number of samples, said number of samples being obtained from a number of subjects, in particular, subjects having cancer. To obtain the threshold, the number of subjects may include subjects having tumors of different molecular subtypes, e.g., subjects having HER2-positive tumors and/or subjects having triple-negative tumors and/or subjects having luminal A tumors and/or subjects having luminal B tumors. The threshold may represent an amount or concentration of the RNA transcript. In one embodiment, the threshold is given as CT (cycle threshold; also referred to as quantification cycle, Cq) value (see below). In one embodiment, the (relative) expression level and expression threshold are expressed as 40-ΔCT or 40-ΔΔCT values (see below).

The term "subject", as used herein, relates to any organism such as vertebrate, particularly any mammal, including both a human and another mammal, e.g. an animal such as a rodent, a rabbit, or a monkey. The rodent may be a mouse, rat, hamster, guinea pig, or chinchilla. Preferably, the subject is a human. In one embodiment, a subject is a subject with or suspected of having a disease, in particular cancer, also designated "patient" herein. For the determination of the mean cut-off value, at least two subjects, preferably at least 5, at least 10, at least 20, at least 30, at least 40, at least 50, at least 60, at least 70, at least 80, at least 90, at least 100, at least 200, at least 300, at least 400, at least 500, at least 600, at least 700, at least 800, at least 900, at least 1000, at least 1500, or at least 2000 subjects, are tested.

As various clinical studies have already been conducted with the gene markers used in accordance with the present invention, a concordance study in a training-testing setting will be sufficient for the definition and validation of a clinical cut-off/threshold for dichotomization of quantitative results in "expression-positive" or "expression-negative". Thus, in one embodiment, the cut-off/threshold is defined based on one or more previous clinical studies. Moreover, additional clinical studies may be conducted for the establishment and validation of the cut-off/threshold. The cut-off/threshold may be determined/defined by techniques known in the art.

In one embodiment, the cut-off/threshold is determined/defined on the basis of clinicopathological parameters, such as IHC-ISH, and/or the data for overall survival (OS), disease-specific survival (DSS), and progression-free survival (PFS), in training cohorts by partitioning tests (e.g., SAS Software JMP^{®} 9.0.0).

In one embodiment, the 40-ΔCT value is calculated as follows: 40 - [CT of the respective biomarker (e.g., HER2, ESR1, PGR and/or Ki67) of a patient sample - CT of a reference gene (e.g., CALM2) of a patient sample] (= calculation method 1). If more than one reference gene is used, the 40-ΔCT value is calculated as follows: 40 - (CT of the respective biomarker of a patient sample - mean CT of selected reference genes of a patient sample) (= calculation method 2). Alternatively, a 40-ΔΔCT value can be used, wherein the 40-ΔΔCT can be calculated as follows: ΔΔCT = 40 - [(CT biomarker of a patient sample - CT biomarker of a reference sample) - (CT reference gene of patient sample - CT reference gene of a reference sample)] (= calculation method 3); e.g., 40-ΔΔCT = 40 - [(CT Ki67 patient sample - CT Ki67 reference sample) - (CT CALM2 of a patient sample - CT CALM2 of a reference sample)]. In one embodiment, CALM2 is used as reference gene.

In another embodiment, the relative expression level of the biomarkers is given as a 40-ΔΔCT value, which is calculated as follows: 40 - [(CT biomarker of a patient sample - CT reference gene of the patient sample) - (CT biomarker of a control sample - CT reference gene of the control sample)] (= calculation method 4); e.g., 40-ΔΔCT = 40 - [(CT Ki67 patient sample - CT Mean CombRef patient sample) - (CT Ki67 control sample - CT Mean CombRef control sample)]. In one embodiment, the CT is the median CT. The CT of the reference gene can be the CT of a single reference gene or the mean CT of two or more reference genes (referred to as Mean CombRef). Preferably, the same control sample (also referred to as calibrator) is used in all analyses and leads to the same RT-qPCR or qPCR results. In one embodiment, the control sample is a cell line RNA, an *in vitro* transcribed artificial RNA or an equimolar mixture of DNA oligonucleotides, representing the biomarker mRNA or cDNA or the biomarker amplicon or a part of the biomarker amplicon with a constant ratio. In one embodiment, CALM2 and/or B2M are used as reference genes and a positive control (e.g., *in vitro* transcribed artificial RNA) is used as control sample (calibrator).

In an exemplary embodiment, the mean cut-off value is given as a 40-ΔΔCT value according to calculation method 4, wherein the mean cut-off value for HER2 is a 40-ΔΔCT value of 40.90, the mean cut-off value for ESR1 is a 40-ΔΔCT value of 35.12, the mean cut-off value for PGR is a 40-ΔΔCT value of 31.24 and/or the mean cut-off value for Ki67 is a 40-ΔΔCT value of 36.12, e.g., on a Versant kPCR Instrument AD module (Siemens).

In another exemplary embodiment, the mean cut-off value is given as a 40-ΔΔCT value according to calculation method 4, wherein the mean cut-off value for HER2 is a 40-ΔΔCT value of 41.10, the mean cut-off value for ESR1 is a 40-ΔΔCT value of 36.12, the mean cut-off value for PGR is a 40-ΔΔCT value of 32.80 and/or the mean cut-off value for Ki67 is a 40-ΔΔCT value of 36.52, 36.65, 37.69 or 38.97, e.g., on a Roche LightCycler 480 instrument II.

In one embodiment, the above mean cut-off value for Ki67 is a 40-ΔΔCT value of 36.52, wherein grade 3 tumors have a 40-ΔΔCT value equal to or above 36.52 and grade 1 and 2 tumors have a 40-ΔΔCT value below 36.52. In one embodiment the mean cut-off value for Ki67 is a 40-ΔΔCT value of 36.65, wherein a 40-ΔΔCT value equal to or above 36.65 indicates a T1 stage or higher stage tumor and a 40-ΔΔCT value below 36.65 indicates a Ta stage tumor. In one embodiment, the mean cut-off value for Ki67 is a 40-ΔΔCT value of 37.69, wherein a 40-ΔΔCT value equal to or above 37.69 indicates an increased risk of cancer progression. In one embodiment, the mean cut-off value for Ki67 is a 40-ΔΔCT value of 38.97, wherein a 40-ΔΔCT value equal to or above 38.97 indicates an increased risk for cancer-specific death.

In one another exemplary embodiment, the mean cut-off value is given as a 40-ΔΔCT value according to calculation method 4, wherein the mean cut-off value for HER2 is a 40-ΔΔCT value of 40.10.

In one embodiment, steps (a), (b), (c) and (d) are performed in random order. In a preferred embodiment, step (a) is performed first, i.e., before steps (b), (c) and (d). In one embodiment, step (d) is performed after steps (a), (b) and (c). In one embodiment, step (a) is performed before step (b), step (b) is performed before step (c), and step (c) is performed before step (d).

In one embodiment, an expression level of RNA transcript of HER2 which is higher than a defined expression threshold of RNA transcript of HER2 identifies the molecular subtype of the tumor as HER2-positive.

In one embodiment, the molecular subtype of the tumor is HER2-positive and an expression level of RNA transcript of ESR1 which is higher than a defined expression threshold of RNA transcript of ESR1 indicates a positive prognosis, wherein, preferably, the positive prognosis comprises an increased probability of one or more of recurrence-free survival (RFS), progression-free survival (PFS), disease-specific survival (DSS), and distant recurrence-free survival, preferably RFS and/or PFS. In one embodiment, the molecular subtype of the tumor is HER2-positive and an expression level of RNA transcript of ESR1 which is lower than a defined expression threshold of RNA transcript of ESR1 indicates a negative prognosis, wherein, preferably, the negative prognosis comprises a reduced probability of one or more of recurrence-free survival (RFS), progression-free survival (PFS), disease-specific survival (DSS), and distant recurrence-free survival, preferably RFS and/or PFS. In one embodiment, the bladder cancer is NMIBC.

In one embodiment, the molecular subtype HER2-positive with an expression level of RNA transcript of ESR1 which is higher than a defined expression threshold of RNA transcript of ESR1 is associated with a probability of RFS and/or PFS 5 years after treatment which is at least 20%, 25% or 30% higher than for the molecular subtype HER2-positive with an expression level of RNA transcript of ESR1 which is lower than a defined expression threshold of RNA transcript of ESR1.

In one embodiment,
- an expression level of RNA transcript of HER2 which is lower than a defined expression threshold of RNA transcript of HER2;
- an expression level of RNA transcript of ESR1 which is lower than a defined expression threshold of RNA transcript of ESR1;
- an expression level of RNA transcript of PGR which is lower than a defined expression threshold of RNA transcript of PGR; and
- an expression level of RNA transcript of Ki67 which is lower or higher than a defined expression threshold of RNA transcript of Ki67
identify the molecular subtype of the tumor as triple-negative.

In one embodiment, the molecular subtype is luminal A or luminal B.

In one embodiment,
- an expression level of RNA transcript of HER2 which is lower than a defined expression threshold of RNA transcript of HER2;
- an expression level of RNA transcript of ESR1 which is higher than a defined expression threshold of RNA transcript of ESR1;
- an expression level of RNA transcript of PGR which is higher than a defined expression threshold of RNA transcript of PGR; and
- an expression level of RNA transcript of Ki67 which is higher than a defined expression threshold of RNA transcript of Ki67
identify the molecular subtype of the tumor as luminal B.

In one embodiment,
- an expression level of RNA transcript of HER2 which is lower than a defined expression threshold of RNA transcript of HER2;
- an expression level of RNA transcript of ESR1 which is higher than a defined expression threshold of RNA transcript of ESR1;
- an expression level of RNA transcript of PGR which is higher than a defined expression threshold of RNA transcript of PGR; and
- an expression level of RNA transcript of Ki67 which is lower than a defined expression threshold of RNA transcript of Ki67
identify the molecular subtype of the tumor as luminal A.

In one embodiment,
- an expression level of RNA transcript of HER2 which is lower than a defined expression threshold of RNA transcript of HER2;
- an expression level of RNA transcript of ESR1 which is higher than a defined expression threshold of RNA transcript of ESR1;
- an expression level of RNA transcript of PGR which is lower than a defined expression threshold of RNA transcript of PGR; and
- an expression level of RNA transcript of Ki67 which is lower or higher than a defined expression threshold of RNA transcript of Ki67
identify the molecular subtype of the tumor as luminal B.

In one embodiment,
- an expression level of RNA transcript of HER2 which is lower than a defined expression threshold of RNA transcript of HER2;
- an expression level of RNA transcript of ESR1 which is lower than a defined expression threshold of RNA transcript of ESR1;
- an expression level of RNA transcript of PGR which is higher than a defined expression threshold of RNA transcript of PGR; and
- an expression level of RNA transcript of Ki67 which is higher than a defined expression threshold of RNA transcript of Ki67
identify the molecular subtype of the tumor as luminal B.

In one embodiment,
- an expression level of RNA transcript of HER2 which is lower than a defined expression threshold of RNA transcript of HER2;
- an expression level of RNA transcript of ESR1 which is lower than a defined expression threshold of RNA transcript of ESR1;
- an expression level of RNA transcript of PGR which is higher than a defined expression threshold of RNA transcript of PGR; and
- an expression level of RNA transcript of Ki67 which is lower than a defined expression threshold of RNA transcript of Ki67
identify the molecular subtype of the tumor as luminal A.

In one embodiment, the expression level of RNA transcript is determined by reverse transcription (RT) quantitative PCR (RT-qPCR). As RNA cannot be directly amplified in PCR, it must be reverse transcribed into cDNA using the enzyme reverse transcriptase. For this purpose, a one-step RT-qPCR can be utilized, which combines the reactions of reverse transcription with DNA amplification by PCR in the same reaction. In one-step RT-qPCR, the RNA template is mixed in a reaction mix containing reverse transcriptase, DNA polymerase, primers and probes, dNTPs, salts and detergents. In a first PCR step, the target RNA is reverse transcribed by reverse transcriptase using the target-specific reverse primers. Afterwards, the cDNA is amplified using the primers/probes and DNA polymerase.

In one embodiment, the quantitative PCR is fluorescence-based quantitative real-time PCR. The fluorescence-based quantitative real-time PCR comprises the use of a fluorescently labeled probe. Preferably, the fluorescently labeled probe consists of an oligonucleotide labeled with both a fluorescent reporter dye and a quencher dye (= dual-label probe). Suitable fluorescent reporter and quencher dyes/moieties are known to a person skilled in the art and include, but are not limited to the reporter dyes/moieties 6-FAM^{™}, JOE^{™}, Cy5^{®}, Cy3^{®} and the quencher dyes/moieties dabcyl, TAMRA^{™}, BHQ^{™}-1, -2 or -3. Amplification of the probe-specific product causes cleavage of the probe (= amplification-mediated probe displacement), thereby generating an increase in reporter fluorescence. The increase of fluorescence in the reaction is directly proportional to the increase of target amplificates. By using the LightCycler 480 II system (Roche) or the Versant kPCR system (Siemens) or the Mx3005P system (Agilent Technologies) or equivalent real-time instruments for detection of fluorescence originating from the probe, one can measure the increase in fluorescence in real-time. Analysis output is a CT value of each target. The CT (cycle threshold; also referred to as quantification cycle, Cq) value is determined by the number of PCR amplification cycles, after which the fluorescence signal of the probe exceeds a certain background signal, wherein the CT value is a measure for the amount of target molecules in the sample before the PCR amplification. Preferably, CT-values are further analyzed with appropriate software (e.g., Microsoft Excel^{™}) or statistical software packages (e.g., SAS JMP^{®} 9.0.0, GraphPad Prism4, Genedata Expressionist^{™}). The CT value can either be converted to an absolute target molecule amount (e.g., ng/µl or molecules/µl) based on the CT results of a standard curve with known target concentrations. Alternatively, the target amount can be reported as x-fold decreased or increased amount based on a reference (= ΔCT). Low ΔCT values (small difference) indicate higher amounts of target relative to the reference compared to high ΔCT (big difference). It is suitable to re-calculate the ΔCT by subtracting it from a fixed value (such as the number of PCR cycles, e.g. 40). The result is a value with direct correlation to target amount (high value = high amount) and expressed as 40-ΔCT values, wherein one integer refers to a doubling of the target amount (e.g., a value of 34 indicates an amount which is twice as much as that with a value of 33). Depending on the desired reproducibility and precision of the system, it is possible to panel multiple reference assays or to re-calculate/normalize the ΔCT of the sample with the ΔCT of a calibrator (1 point calibration; Pfaffl (2001), Nucleic Acid Res., 29(9):e45). By using different fluorophores for specific probes it is also possible to multiplex different target assays in the same reaction. During PCR, each target in the multiplex is amplified in parallel, but separately detected utilizing the different fluorescent emission.

Preferably, primers for use in accordance with the present invention have a length of 15 to 30 nucleotides, in particular deoxyribonucleotides. In one embodiment, the primers are designed so as to (1) be specific for the target mRNA-sequence (e.g., HER2, ESR1, PGR or Ki67), (2) provide an amplicon size of less than 120 bp (preferably less than 100 bp), (3) detect all known protein-encoding splicing variants, (4) not include known polymorphisms (e.g., single nucleotide polymorphisms, SNPs), (5) be mRNA-specific (consideration of exons/introns; preferably no amplification of DNA), (6) have no tendency to dimerize and/or (7) have a melting temperature Tₘ in the range of from 58°C to 62°C (preferably, Tₘ is approximately 60°C).

As used herein, the term "nucleotide" includes native (naturally occurring) nucleotides, which include a nitrogenous base selected from the group consisting of adenine (A), thymidine (T), cytosine (C), guanine (G) and uracil (U), a sugar selected from the group of ribose, arabinose, xylose, and pyranose, and deoxyribose (the combination of the base and sugar generally referred to as a "nucleoside"), and one to three phosphate groups, and which can form phosphodiester internucleosidyl linkages. Further, as used herein, "nucleotide" refers to nucleotide analogues. As used herein, "nucleotide analogue" shall mean an analogue of A, G, C, T or U (that is, an analogue of a nucleotide comprising the base A, G, C, T or U) which is recognized by DNA or RNA polymerase (whichever is applicable) and incorporated into a strand of DNA or RNA (whichever is appropriate). Examples of such nucleotide analogues include, without limitation, 5-propynyl pyrimidines (i.e., 5-propynyl-dTTP and 5-propynyl-dCTP), 7-deaza purines (i.e., 7-deaza-dATP and 7-deaza-dGTP), aminoallyl-dNTPs, biotin-AA-dNTPs, 2-amino-dATP, 5-methyl-dCTP, 5-iodo-dUTP, 5-bromo-dUTP, 5-fluoro-dUTP, N4-methyl-dCTP, 2-thio-dTTP, 4-thio-dTTP and alpha-thio-dNTPs. Also included are labelled analogues, e.g. fluorescent analogues such as DEAC-propylenediamine *(*PDA*)*-ATP, analogues based on morpholino nucleoside analogues as well as locked nucleic acid (LNA) analogues.

The wording "specific for the target mRNA-sequence", as used in connection with primers for use in accordance with the present invention, is meant to refer to the ability of the primer to hybridize (i.e. anneal) to the cDNA of the target mRNA-sequence under appropriate conditions of temperature and solution ionic strength, in particular PCR conditions. The conditions of temperature and solution ionic strength determine the stringency of hybridization. Hybridization requires that the two nucleic acids (i.e. primer and cDNA) contain complementary sequences, although depending on the stringency of the hybridization, mismatches between bases are possible. In one embodiment, "appropriate conditions of temperature and solution ionic strength" refer to a temperature in the range of from 58°C to 62°C (preferably a temperature of approximately 60°C) and a solution ionic strength commonly used in PCR reaction mixtures. In one embodiment, the sequence of the primer is 80%, preferably 85%, more preferably 90%, even more preferably 95%, 96%, 97%, 98%, 99% or 100% complementary to the corresponding sequence of the cDNA of the target mRNA-sequence, as determined by sequence comparison algorithms known in the art.

In one embodiment, the primer hybridizes to the cDNA of the target mRNA-sequence under stringent or moderately stringent hybridization conditions. "Stringent hybridization conditions", as defined herein, involve hybridizing at 68°C in 5x SSC/5x Denhardt's solution/1,0% SDS, and washing in 0,2x SSC/0,1 % SDS at room temperature, or involve the art-recognized equivalent thereof (e.g., conditions in which a hybridization is carried out at 60°C in 2,5x SSC buffer, followed by several washing steps at 37°C in a low buffer concentration, and remains stable). "Moderately stringent hybridization conditions", as defined herein, involve including washing in 3x SSC at 42°C, or the art-recognized equivalent thereof. The parameters of salt concentration and temperature can be varied to achieve the optimal level of identity between the primer and the target nucleic acid. Guidance regarding such conditions is available in the art, for example, by J. Sambrook et al. eds., 2000, Molecular Cloning: A Laboratory Manual, 3rd Edition, Cold Spring Harbor Laboratory Press, Cold Spring Harbor; and Ausubel et al. eds., 1995, Current Protocols in Molecular Biology, John Wiley and Sons, N.Y..

In one embodiment, the method comprises the use of ESR1 -specific primers having a length of 15 to 30 nucleotides and comprising at least 10 contiguous nucleotides of the sequences of SEQ ID NOs: 1 and 2, and/or HER2-specific primers having a length of 15 to 30 nucleotides and comprising at least 10 contiguous nucleotides of the sequences of SEQ ID NOs: 4 and 5, and/or Ki67-specific primers having a length of 15 to 30 nucleotides and comprising at least 10 contiguous nucleotides of the sequences of SEQ ID NOs: 7 and 8, and/or PGR-specific primers having a length of 15 to 30 nucleotides and comprising at least 10 contiguous nucleotides of the sequences of SEQ ID NOs: 10 and 11, and/or RACGAP1-specific primers having a length of 15 to 30 nucleotides and comprising at least 10 contiguous nucleotides of the sequences of SEQ ID NOs: 13 and 14. In one embodiment, the specific primers comprise at least 15 contiguous nucleotides of the sequences indicated above.

In one embodiment, the method comprises the use of ESR1-specific primers comprising or having the sequences of SEQ ID NOs: 1 and 2, and/or HER2-specific primers comprising or having the sequences of SEQ ID NOs: 4 and 5, and/or Ki67-specific primers comprising or having the sequences of SEQ ID NOs: 7 and 8, and/or PGR-specific primers comprising or having the sequences of SEQ ID NOs: 10 and 11, and/or RACGAP1-specific comprising or having the sequences of SEQ ID NOs: 13 and 14.

Preferably, probes for use in accordance with the present invention have a length of 20 to 35 nucleotides, in particular deoxyribonucleotides. In one embodiment, the probes are designed so as to (1) be specific for the target mRNA-sequence (e.g., HER2, ESR1, PGR or Ki67), (2) not include known polymorphisms (e.g., single nucleotide polymorphisms, SNPs) and/or (3) have a melting temperature Tₘ, which is approximately 5°C to 8°C higher than the melting temperature Tₘ of the corresponding primer(s).

The wording "specific for the target mRNA-sequence", as used in connection with probes for use in accordance with the present invention, is meant to refer to the ability of the probe to hybridize (i.e. anneal) to the (amplified) cDNA of the target mRNA-sequence under appropriate conditions of temperature and solution ionic strength, in particular PCR conditions. The conditions of temperature and solution ionic strength determine the stringency of hybridization. Hybridization requires that the two nucleic acids (i.e. probe and cDNA) contain complementary sequences, although depending on the stringency of the hybridization, mismatches between bases are possible. In one embodiment, "appropriate conditions of temperature and solution ionic strength" refer to a temperature in the range of from 63°C to 70°C and a solution ionic strength commonly used in PCR reaction mixtures. In one embodiment, the sequence of the probe is 80%, preferably 85%, more preferably 90%, even more preferably 95%, 96%, 97%, 98%, 99% or 100% complementary to the corresponding sequence of the (amplified) cDNA of the target mRNA-sequence, as determined by sequence comparison algorithms known in the art.

In one embodiment, the probe hybridizes to the (amplified) cDNA of the target mRNA-sequence under stringent or moderately stringent hybridization conditions as defined above.

In one embodiment, the method comprises the use of an ESR1-specific probe having a length of 20 to 35 nucleotides and comprising at least 15 contiguous nucleotides of the sequence of SEQ ID NO: 3, and/or a HER2-specific probe having a length of 20 to 35 nucleotides and comprising at least 15 contiguous nucleotides of the sequence of SEQ ID NO: 6, and/or a Ki67-specific probe having a length of 20 to 35 nucleotides and comprising at least 15 contiguous nucleotides of the sequence of SEQ ID NO: 9, and/or a PGR-specific probe having a length of 20 to 35 nucleotides and comprising at least 15 contiguous nucleotides of the sequence of SEQ ID NO: 12, and/or a RACGAP1-specific probe having a length of 20 to 35 nucleotides and comprising at least 15 contiguous nucleotides of the sequence of SEQ ID NO: 15. In one embodiment, the specific probes comprise at least 20 contiguous nucleotides of the sequences indicated above.

In one embodiment, the method comprises the use of an ESR1-specific probe comprising or having the sequence of SEQ ID NO: 3, and/or a HER2-specific probe comprising or having the sequence of SEQ ID NO: 6, and/or a Ki67-specific probe comprising or having the sequence of SEQ ID NO: 9, and/or a PGR-specific probe comprising or having the sequence of SEQ ID NO: 12, and/or a RACGAP1-specific probe comprising or having the sequence of SEQ ID NO: 15.

The probes as defined above are preferably labeled, e.g., with a label selected from a fluorescent label, a fluorescence quenching label, a luminescent label, a radioactive label, an enzymatic label and combinations thereof. Preferably, the probes as defined above are dual-label probes comprising a fluorescence reporter moiety and a fluorescence quencher moiety.

In one embodiment, the expression level is normalized against the (mean) expression level of one or more reference genes in the sample of the tumor. The term "reference gene", as used herein, is meant to refer to a gene which has a relatively invariable level of expression on the RNA transcript/mRNA level in the system which is being examined, i.e. cancer. Such gene may be referred to as housekeeping gene. In one embodiment, the one or more reference genes are selected from the group comprising CALM2, B2M, RPL37A, GUSB, HPRT1 and GAPDH, preferably CALM2 and/or B2M. Other suitable reference genes are known to a person skilled in the art.

As used herein, CALM2 refers to calmodulin-2, phosphorylase kinase, delta (Ref.Seq. (mRNA): NM_001743), B2M refers to beta-2 microglobulin (Ref.Seq. (mRNA): NM_004048), RPL37A refers to 60S ribosomal protein L37a (Ref.Seq. (mRNA): NM_000998), GUSB refers to beta-glucuronidase (Ref.Seq. (mRNA): NM_000181), HPRT1 refers to hypoxanthine-phosphoribosyl-transferase 1 (Ref.Seq. (mRNA): NM_000194) and GAPDH refers to glycerinaldehyde-3-phosphate-dehydrogenase (Ref.Seq. (mRNA): NM_002046).

The term "non-reference gene", as used herein is meant to refer to a gene which has a variable level of expression on the RNA transcript/mRNA level in the system which is being examined, i.e. cancer, and thus can be used, e.g., for the subtyping of tumors/cancers and/or the assessment of cancer progression. In one embodiment, the non-reference gene is selected from tumor markers, e.g., those known from the prior art. Non-reference genes that can be used in accordance with the present invention may be bladder-specific or non-bladder-specific genes.

In a further aspect, the invention relates to a method of stratifying a bladder cancer patient for tumor treatment, said method comprising, as a first step, identifying a molecular subtype of a tumor in the cancer patient using the *in vitro* method as defined above and, as a second step, selecting a tumor treatment regimen based on the molecular subtype identified by the *in vitro* method.

"Stratifying a cancer patient for tumor treatment" in accordance with the present invention comprises the allocation of the cancer patient to a patient group having a particular molecular tumor subtype, which then allows the medical practitioner to select the most suitable tumor treatment regimen.

In one embodiment, said method of stratifying a bladder cancer patient for tumor treatment does not comprise any other diagnostic steps, such as histological grading or determining the lymph nodal status, besides the step of identifying the molecular subtype of the tumor in the cancer patient using the *in vitro* method as defined above. In one embodiment, said method does not comprise any steps involving immunohistochemistry (IHC).

In one embodiment, the molecular subtype is selected from the group comprising, preferably consisting of, HER2-positive, triple-negative, luminal A and luminal B.

In one embodiment,
- the molecular subtype is HER2-positive, and the tumor treatment regimen comprises transurethral resection and/or BCG-instillation and/or chemotherapy and/or anti-HER2 therapy and/or administration of antibodies targeting immune checkpoints and/or cystectomy followed by administration of anti-HER2 therapy and/or chemotherapy;
- the molecular subtype is triple-negative, and the tumor treatment regimen comprises transurethral resection and/or BCG-instillation and/or chemotherapy, in particular neoadjuvant chemotherapy, and/or administration of antibodies targeting immune checkpoints and/or cystectomy;
- the molecular subtype is luminal A, and the tumor treatment regimen comprises transurethral resection and/or BCG-instillation and/or cystectomy and/or chemotherapy, in particular adjuvant chemotherapy, and/or (adjuvant) endocrine therapy; and/or
- the molecular subtype is luminal B, and the tumor treatment regimen comprises transurethral resection and/or BCG-instillation and/or endocrine therapy and/or chemotherapy, in particular adjuvant chemotherapy or chemotherapy in the perioperative situation, and/or cystectomy.

In one embodiment,
- the molecular subtype is luminal A, the bladder cancer is NMIBC, and the tumor treatment regimen comprises transurethral resection (TUR) and/or Bacillus Calmette-Guerin (BCG)-instillation, preferably TUR and BCG-instillation;
- the molecular subtype is luminal B, the bladder cancer is NMIBC, and the tumor treatment regimen comprises adjuvant chemotherapy with or without adjuvant endocrine therapy;
- the molecular subtype is HER2-positive, the bladder cancer is NMIBC, and the tumor treatment regimen comprises (neo)adjuvant chemotherapy with or without (neo)adjuvant anti-HER2 therapy;
- the molecular subtype is triple-negative, the bladder cancer is NMIBC, and the tumor treatment regimen comprises neoadjuvant chemotherapy;
- the molecular subtype is luminal A, the bladder cancer is MIBC, and the tumor treatment regimen comprises (i) cystectomy and (ii) adjuvant chemotherapy and/or adjuvant endocrine therapy, preferably adjuvant chemotherapy or adjuvant endocrine therapy;
- the molecular subtype is luminal B, the bladder cancer is MIBC, and the tumor treatment regimen comprises (i) cystectomy and (ii) adjuvant chemotherapy and/or adjuvant endocrine therapy, preferably adjuvant chemotherapy and adjuvant endocrine therapy;
- the molecular subtype is HER2-positive, the bladder cancer is MIBC, and the tumor treatment regimen comprises (i) cystectomy and (ii) adjuvant chemotherapy and/or adjuvant anti-HER2 therapy, preferably adjuvant chemotherapy and adjuvant anti-HER2 therapy; and/or
- the molecular subtype is triple-negative, the bladder cancer is MIBC, and the tumor treatment regimen comprises neoadjuvant chemotherapy with or without subsequent cystectomy.

In one embodiment, the molecular subtype is HER2-positive, the bladder cancer is preferably NMIBC, and the tumor treatment regimen comprises anti-HER2 therapy in combination with endocrine therapy, hormonal therapy and/or chemotherapy, all of which can be in the form of adjuvant or neoadjuvant therapy.

In another embodiment, the molecular subtype is HER2-positive, the bladder cancer is preferably NMIBC, and the tumor treatment regimen comprises instillation therapy, e.g., BCG-instillation.

In one embodiment, the molecular subtype is luminal B, the bladder cancer is preferably NMIBC, and the tumor treatment regimen comprises instillation therapy (e.g., BCG-instillation) and/or neoadjuvant/adjuvant chemotherapy, preferably in combination with endocrine therapy, e.g., with tamoxifen (Nolvadex^{®}), fulvestrant (Faslodex^{®}) or aromatase inhibitors.

The meaning of the term "anti-HER2 therapy" is known to a person skilled in the art. In one embodiment, anti-HER2 therapy comprises the administration of anti-HER2 antibodies, in particular monoclonal anti-HER2 antibodies. Monoclonal anti-HER2 antibodies include trastuzumab (Herceptin^{®}) and pertuzumab (Peijeta^{®}), which may be administered alone or in combination. Trastuzumab is effective only in cancers where HER2 is over-expressed. Other monoclonal antibodies, such as ertumaxomab (Rexomun^{®}), are presently undergoing clinical trials. The anti-HER2 antibodies can further be modified to comprise a therapeutic moiety/agent, such as a cytotoxic agent, a drug (e.g., an immunosuppressant), a chemotherapeutic agent or a radionuclide, or a radioisotope. Thus, if the tumor treatment regimen comprises (a combination of) anti-HER2 therapy and chemotherapy, an anti-HER2 antibody conjugated to a chemotherapeutic agent may be used. A cytotoxin or cytotoxic agent includes any agent that is detrimental to and, in particular, kills cells. Examples include mertansine or emtansine (DM1), taxol, cytochalasin B, gramicidin D, ethidium bromide, emetine, mitomycin, etoposide, tenoposide, vincristine, vinblastine, colchicin, doxorubicin, daunorubicin, dihydroxy anthracin, dione, mitoxantrone, mithramycin, actinomycin D, amanitin, 1-dehydrotestosterone, glucocorticoids, procaine, tetracaine, lidocaine, propranolol, and puromycin and analogs or homologs thereof. In one embodiment, the antibody conjugate is trastuzumab (T)-DM1, e.g., trastuzumab emtansine. Other suitable therapeutic agents for forming antibody conjugates include, but are not limited to, antimetabolites (e.g., methotrexate, 6-mercaptopurine, 6-thioguanine, cytarabine, fludarabin, 5-fluorouracil decarbazine), alkylating agents (e.g., mechlorethamine, thioepachlorambucil, melphalan, carmustine (BSNU) and lomustine (CCNU), cyclophosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine). In a preferred embodiment, the therapeutic agent is a cytotoxic agent or a radiotoxic agent. In another embodiment, the therapeutic agent is an immunosuppressant. In yet another embodiment, the therapeutic agent is GM-CSF. In another preferred embodiment, the therapeutic agent is doxorubicin, cisplatin, bleomycin, sulfate, carmustine, chlorambucil, cyclophosphamide or ricin A. Further therapeutic moieties include therapeutic moieties acting on mRNA and/or protein synthesis. Several inhibitors of transcription are known. For instance, actinomycin D, which is both a transcriptional inhibitor and a DNA damage agent, intercalates within the DNA and thus inhibits the initiation stage of transcription. Flavopiridol targets the elongation stage of transcription. α-Arnanitin binds directly to RNA polymerase II, which leads to the inhibition of both initiation and elongation stages. Anti-HER2 antibodies also can be conjugated to a radioisotope, e.g., iodine-131, yttrium-90 or indium-Ill, to generate cytotoxic radiopharmaceuticals. An alternative to the administration of anti-HER2 antibodies is the administration of small compounds targeting HER2, such as lapatinib (Tykerb^{®} or Tyverb^{®}), afatinib or neratinib. Anti-HER2 therapy may also be supplemented with endocrine therapy (also referred to as anti-hormonal treatment), hormonal therapy, e.g., with progestin, and/or chemotherapy.

Chemotherapy comprises the administration of chemotherapeutic agents. Chemotherapeutic agents according to the invention include cytostatic compounds and cytotoxic compounds. Traditional chemotherapeutic agents act by killing cells that divide rapidly, one of the main properties of most cancer cells. According to the invention, the term "chemotherapeutic agent" includes taxanes, platinum compounds, nucleoside analogs, camptothecin analogs, anthracyclines and anthracycline analogs, etoposide, bleomycin, vinorelbine, cyclophosphamide, antimetabolites, anti-mitotics, and alkylating agents, including the agents disclosed above in connection with antibody conjugates, and combinations thereof. In one embodiment, the chemotherapy is platinum-based, i.e. comprises the administration of platinum-based compounds, e.g., cisplatin. According to the invention a reference to a chemotherapeutic agent is to include any prodrug such as ester, salt or derivative such as a conjugate of said agent. Examples are conjugates of said agent with a carrier substance, e.g., protein-bound paclitaxel such as albumin-bound paclitaxel. Preferably, salts of said agent are pharmaceutically acceptable. Chemotherapeutic agents are often given in combinations, usually for 3-6 months. One of the most common treatments is cyclophosphamide plus doxorubicin (adriamycin; belonging to the group of anthracyclines and anthracycline analogs), known as AC. Sometimes, a taxane drug, such as docetaxel, is added, and the regime is then known as CAT; taxane attacks the microtubules in cancer cells. Another common treatment, which produces equivalent results, is cyclophosphamide, methotrexate, which is an antimetabolite, and fluorouracil, which is a nucleoside analog (CMF). Another standard chemotherapeutic treatment comprises fluorouracil, epirubicin and cyclophosphamide (FEC), which may be supplemented with a taxane, such as docetaxel, or with vinorelbine.

In one embodiment, the molecular subtype is luminal B, and the tumor treatment regimen comprises administration of chemotherapeutic agents. In one embodiment, the molecular subtype is luminal B, and the tumor treatment regimen comprises administration of a taxane, preferably docetaxel. In one embodiment, the taxane is administered in combination with platinum-based chemotherapy.

Endocrine therapy (also referred to as anti-hormonal treatment) targets cancers that require estrogen to continue growing by administration of drugs that either block/down-regulate estrogen and/or progesterone receptors, e.g., tamoxifen (Nolvadex^{®}) or fulvestrant (Faslodex^{®}), or alternatively block the production of estrogen with an aromatase inhibitor, e.g., anastrozole (Arimidex^{®}) or letrozole (Femara^{®}). Aromatase inhibitors, however, are only suitable for postmenopausal patients. This is because the active aromatase in postmenopausal women is different from the prevalent form in premenopausal women, and therefore these agents are ineffective in inhibiting the predominant aromatase of premenopausal women.

In another aspect, the invention relates to a method of treatment of bladder cancer, the method comprising, as a first step, stratifying a bladder cancer patient for tumor treatment using the method as defined above and, as a second step, providing the selected tumor treatment regimen to the bladder cancer patient. The tumor treatment regimen is selected based on the molecular subtype identified by the *in vitro* method as defined above.

The first step and the second step of said method may be performed separately from each other, in terms of time and/or location. The first step may, for example, result in the issuance of treatment guidelines, which are used for performing the second step at a different time and/or location. The first step may also be immediately followed by the second step.

In one embodiment, said method comprises using quantitative results obtained by the *in vitro* method as defined above for direct decision-making in favor of or against adjuvant/neoadjuvant chemotherapy.

In another aspect, the present invention relates to a method of treatment of bladder cancer, wherein the bladder cancer is characterized by a molecular subtype as defined herein, and wherein the method comprises providing a tumor treatment regimen that is selected based on the molecular subtype.

In yet another aspect, the invention relates to the use of a kit for identifying a molecular subtype of a tumor in a bladder cancer patient by means of reverse transcription (RT) quantitative PCR (RT-qPCR), said kit comprising at least one pair of primers and at least one probe that are specific for a gene selected from the group consisting of HER2, ESR1, PGR, Ki67 and RACGAP1.

In one embodiment, the kit comprises specific pairs of primers and specific probes for at least two, three or four genes selected from the group consisting of HER2, ESR1, PGR, Ki67 and RACGAP1.

In one embodiment, the kit comprises:
- at least one pair of HER2-specific primers and at least one HER2-specific probe;
- at least one pair of ESR1-specific primers and at least one ESR1-specific probe; and, optionally,
- at least one pair of Ki67-specific primers and at least one Ki67-specific probe; and/or
- at least one pair of RACGAP 1 -specific primers and at least one RACGAP1 -specific probe.

In one embodiment, the kit comprises:
- at least one pair of HER2-specific primers and at least one HER2-specific probe;
- at least one pair of ESR1 -specific primers and at least one ESR1 -specific probe; and
- at least one pair of Ki67-specific primers and at least one Ki67-specific probe.

In one embodiment, the kit further comprises:
- at least one pair of PGR-specific primers and at least one PGR-specific probe.

In one embodiment, the invention relates to the use of a kit for identifying a molecular subtype of a tumor in a bladder cancer patient by means of reverse transcription (RT) quantitative PCR (RT-qPCR), said kit comprising:
- at least one pair of HER2-specific primers and at least one HER2-specific probe;
- at least one pair of ESR1-specific primers and at least one ESR1-specific probe;
- at least one pair of PGR-specific primers and at least one PGR-specific probe; and
- at least one pair of Ki67-specific primers and at least one Ki67-specific probe; and/or
- at least one pair of RACGAP 1 -specific primers and at least one RACGAP 1 -specific probe.

In one embodiment, the kit comprises:
- at least one pair of HER2-specific primers and at least one HER2-specific probe;
- at least one pair of ESR1 -specific primers and at least one ESR1 -specific probe;
- at least one pair of PGR-specific primers and at least one PGR-specific probe; and
- at least one pair of Ki67-specific primers and at least one Ki67-specific probe.

In one embodiment, the kit comprises one or more sets of primers and probe that are specific for additional non-reference genes.

In one embodiment, the kit does not comprise more than three, two or one set of primers and probe that are specific for additional non-reference genes.

In one embodiment, the kit does not comprise any sets of primers and probe that are specific for additional non-reference genes. In other words, no set of primers and probe specific for a gene other than HER2, ESR1, PGR and, optionally, at least one gene selected from Ki67 and RACGAP1, and, optionally, one or more reference genes is comprised in the kit. In one embodiment, no set of primers and probe specific for a gene other than HER2, ESR1, PGR, Ki67 and, optionally, one or more reference genes is comprised in the kit. In another embodiment, no set of primers and probe specific for a gene other than HER2, ESR1 and, optionally, at least one gene selected from Ki67 and RACGAP 1, preferably Ki67, and, optionally, one or more reference genes is comprised in the kit. In yet another embodiment, no set of primers and probe specific for a gene other than HER2, ESR1 and, optionally, one or more reference genes is comprised in the kit.

In one embodiment, the kit comprises a maximum of 7, preferably 6, more preferably 5, even more preferably 4 different sets of primers and probe specific for non-reference genes.

In one embodiment, the quantitative PCR is fluorescence-based quantitative real-time PCR.

In one embodiment, detection of the probe is based on amplification-mediated probe displacement. In one embodiment, the probe is a dual-label probe comprising a fluorescence reporter moiety and a fluorescence quencher moiety.

In one embodiment, the kit further comprises a reverse transcriptase and a DNA polymerase. In one embodiment, the reverse transcriptase and the DNA polymerase are provided in the form of an enzyme-mix which allows a one-step reverse transcription (RT) quantitative PCR (RT-qPCR).

In one embodiment, the kit further comprises at least one pair of reference gene-specific primers and at least one reference gene-specific probe. In one embodiment, the reference gene is one or more selected from the group comprising CALM2, B2M, RPL37A, GUSB, HPRT1 and GAPDH, preferably CALM2 and/or B2M.

In one embodiment, the kit further comprises at least one control RNA sample. In one embodiment, the at least one control RNA sample is used as a positive control and/or a control sample (calibrator), wherein, preferably, the at least one control RNA sample comprises synthetic mRNA coding for one or more gene products (or parts thereof) of one or more genes selected from the group comprising HER2, ESR1, PGR, Ki67, RACGAP1 and one or more reference genes. In one embodiment, the one or more reference genes are selected from the group comprising CALM2, B2M, RPL37A, GUSB, HPRT1 and GAPDH, preferably CALM2 and/or B2M.

In one embodiment, the kit may further comprise a DNase and a DNase reaction buffer.

Preferably, the primers and probes are as defined further above in connection with the *in vitro* method of the present invention.

In one embodiment, the primers provide an amplicon size of less than 120 bp, preferably less than 100 bp.

In one embodiment, the ESR1-specific primers have a length of 15 to 30 nucleotides and comprise at least 10 contiguous nucleotides of the sequences of SEQ ID NOs: 1 and 2, and/or the HER2-specific primers have a length of 15 to 30 nucleotides and comprise at least 10 contiguous nucleotides of the sequences of SEQ ID NOs: 4 and 5, and/or the Ki67-specific primers have a length of 15 to 30 nucleotides and comprise at least 10 contiguous nucleotides of the sequences of SEQ ID NOs: 7 and 8, and/or the PGR-specific primers have a length of 15 to 30 nucleotides and comprise at least 10 contiguous nucleotides of the sequences of SEQ ID NOs: 10 and 11, and/or the RACGAP1-specific primers have a length of 15 to 30 nucleotides and comprise at least 10 contiguous nucleotides of the sequences of SEQ ID NOs: 13 and 14. In one embodiment, the specific primers comprise at least 15 contiguous nucleotides of the sequences indicated above.

In one embodiment, the ESR1-specific primers comprise or have the sequences of SEQ ID NOs: 1 and 2, and/or the HER2-specific primers comprise or have the sequences of SEQ ID NOs: 4 and 5, and/or the Ki67-specific primers comprise or have the sequences of SEQ ID NOs: 7 and 8, and/or the PGR-specific primers comprise or have the sequences of SEQ ID NOs: 10 and 11, and/or the RACGAP1-specific comprise or have the sequences of SEQ ID NOs: 13 and 14.

In one embodiment, the ESR1-specific probe has a length of 20 to 35 nucleotides and comprises at least 15 contiguous nucleotides of the sequence of SEQ ID NO: 3, and/or the HER2-specific probe has a length of 20 to 35 nucleotides and comprises at least 15 contiguous nucleotides of the sequence of SEQ ID NO: 6, and/or the Ki67-specific probe has a length of 20 to 35 nucleotides and comprises at least 15 contiguous nucleotides of the sequence of SEQ ID NO: 9, and/or the PGR-specific probe has a length of 20 to 35 nucleotides and comprises at least 15 contiguous nucleotides of the sequence of SEQ ID NO: 12, and/or the RACGAP1-specific probe has a length of 20 to 35 nucleotides and comprises at least 15 contiguous nucleotides of the sequence of SEQ ID NO: 15. In one embodiment, the specific probes comprise at least 20 contiguous nucleotides of the sequences indicated above.

In one embodiment, the ESR1-specific probe comprises or has the sequence of SEQ ID NO: 3, and/or the HER2-specific probe comprises or has the sequence of SEQ ID NO: 6, and/or the Ki67-specific probe comprises or has the sequence of SEQ ID NO: 9, and/or the PGR-specific probe comprises or has the sequence of SEQ ID NO: 12, and/or the RACGAP 1 -specific probe comprises or has the sequence of SEQ ID NO: 15.

Preferably, the probes as defined above are dual-label probes comprising a fluorescence reporter moiety and a fluorescence quencher moiety.

As used herein, the term "kit of parts (in short: kit)" refers to an article of manufacture comprising one or more containers and, optionally, a data carrier. Said one or more containers may be filled with one or more of the above mentioned means or reagents. Additional containers may be included in the kit that contain, e.g., diluents, buffers and further reagents such as dNTPs. Said data carrier may be a non-electronical data carrier, e.g., a graphical data carrier such as an information leaflet, an information sheet, a bar code or an access code, or an electronical data carrier such as a compact disk (CD), a digital versatile disk (DVD), a microchip or another semiconductor-based electronical data carrier. The access code may allow the access to a database, e.g., an internet database, a centralized, or a decentralized database. Said data carrier may comprise instructions for the use of the kit in the methods of the invention. The data carrier may comprise a threshold value or reference level of RNA transcript, e.g., mRNA. In case that the data carrier comprises an access code which allows the access to a database, said threshold value or reference level is deposited in this database. In addition, the data carrier may comprise information or instructions on how to carry out the methods of the present invention. For example, the kit may contain an instruction for use (IFU) and/or a guideline indicating the molecular subtypes as defined herein and/or the tumor treatment regimens to be selected based on the molecular subtypes as defined herein.

In another aspect, the present invention relates to a kit as defined above. Such kit may be referred to herein as BladderTyper kit, in particular as BladderTyper RT-qPCR kit.

In another aspect, the present invention relates to the use of a kit as defined above for assessing a bladder cancer patient's probability for disease-specific survival and/or risk for distant metastasis, preferably a bladder cancer patient's probability for disease-specific survival.

In another aspect, the present invention relates to the use of a pair of primers as defined herein and/or a probe as defined herein for identifying a molecular subtype of a tumor in a bladder cancer patient, e.g., in a method as defined herein, wherein the pair of primers and/or probe is specific for a gene selected from the group consisting of HER2, ESR1, PGR, Ki67 and RACGAP1.

In one embodiment, the molecular subtype is as defined herein.

In one embodiment, the probe is a dual-label probe comprising a fluorescence reporter moiety and a fluorescence quencher moiety.

In yet another aspect, the present invention relates to the use of a pair of primers as defined herein and/or a probe as defined herein for manufacturing of a kit for identifying a molecular subtype of a tumor in a bladder cancer patient by means of reverse transcription (RT) quantitative PCR (RT-qPCR), wherein the pair of primers and/or probe is specific for a gene selected from the group consisting of HER2, ESR1, PGR, Ki67 and RACGAP1.

In one embodiment, the kit is as defined herein.

In one embodiment, the molecular subtype is as defined herein.

In one embodiment, the probe is a dual-label probe comprising a fluorescence reporter moiety and a fluorescence quencher moiety.

In a further aspect, the invention relates to the use of the expression level of RNA transcript of ESR1 and/or the expression level of RNA transcript of PGR as prognostic biomarker(s) for bladder cancer.

In one embodiment, the expression level of RNA transcript of ESR1 or the expression level of RNA transcript of PGR is used as a singular prognostic biomarker.

The term "prognostic marker", as used herein, refers to a marker that provides information on the likely course of the respective disease (here: bladder cancer) in a treated or untreated patient. In one embodiment, the prognosis comprises one or more of disease-specific survival (DSS), recurrence-free survival (RFS), progression-free survival (PFS) and distant recurrence-free survival. In one embodiment, the prognosis comprises DSS. The term "singular prognostic biomarker", as used herein, means that no additional prognostic marker is used/analyzed for the prognosis.

In one embodiment,
- an expression level of RNA transcript of ESR1 which is higher than a defined expression threshold of RNA transcript of ESR1 indicates a positive prognosis; and/or
- an expression level of RNA transcript of PGR which is higher than a defined expression threshold of RNA transcript of PGR indicates a positive prognosis.

In one embodiment, the positive prognosis comprises an increased probability of one or more of prolonged disease-specific survival (DSS), recurrence-free survival (RFS), progression-free survival (PFS) and distant recurrence-free survival, preferably DSS.

In one embodiment,
- an expression level of RNA transcript of ESR1 which is lower than a defined expression threshold of RNA transcript of ESR1 indicates a negative prognosis; and/or
- an expression level of RNA transcript of PGR which is lower than a defined expression threshold of RNA transcript of PGR indicates a negative prognosis.

In one embodiment, the negative prognosis comprises a reduced probability of one or more of prolonged disease-specific survival (DSS), recurrence-free survival (RFS), progression-free survival (PFS) and distant recurrence-free survival, preferably DSS.

In one embodiment, an expression level of RNA transcript of ESR1 or PGR which is higher than a defined expression threshold of RNA transcript of ESR1 or PGR, respectively, is associated with a probability of DSS 10 years after treatment which is at least 25%, preferably at least 30%, more preferably at least 35% higher than the probability of DSS 10 years after treatment associated with an expression level of RNA transcript of ESR1 or PGR which is lower than a defined expression threshold of RNA transcript of ESR1 or PGR, respectively.

In one embodiment, the bladder cancer, preferably NMIBC, is of the HER2-positive molecular subtype and an expression level of RNA transcript of ESR1 which is higher than a defined expression threshold of RNA transcript of ESR1 indicates a positive prognosis, wherein, preferably, the positive prognosis comprises an increased probability of one or more of recurrence-free survival (RFS), progression-free survival (PFS), disease-specific survival (DSS), and distant recurrence-free survival, preferably RFS and/or PFS.

In one embodiment, the bladder cancer, preferably NMIBC, is of the HER2-positive molecular subtype and an expression level of RNA transcript of ESR1 which is lower than a defined expression threshold of RNA transcript of ESR1 indicates a negative prognosis, wherein, preferably, the negative prognosis comprises a reduced probability of one or more of recurrence-free survival (RFS), progression-free survival (PFS), disease-specific survival (DSS), and distant recurrence-free survival, preferably RFS and/or PFS.

In one embodiment, the molecular subtype HER2-positive with an expression level of RNA transcript of ESR1 which is higher than a defined expression threshold of RNA transcript of ESR1 is associated with a probability of RFS and/or PFS 5 years after treatment which is at least 20%, 25% or 30% higher than for the molecular subtype HER2-positive with an expression level of RNA transcript of ESR1 which is lower than a defined expression threshold of RNA transcript of ESR1.

In yet a further aspect, the present invention relates to the use of the expression level of RNA transcript of Ki67 as a biomarker for the grading and/or staging of a bladder cancer tumor, to a method for the grading and/or staging of a bladder cancer tumor comprising determining the expression level of RNA transcript of Ki67 in a sample of the tumor as well as to the use of a kit comprising at least one pair of Ki67-specific primers and at least one Ki67-specific probe for the grading and/or staging of a bladder cancer tumor.

The present invention overcomes major disadvantages of the current standard and state of the art diagnostic methods based on immunohistochemistry as well as more recent methods. The present invention allows reliable molecular subtyping of tumors, which then allows the medical practitioner to select the most suitable tumor treatment regimen.

Thanks to the preferred order of the assessment of the four markers (preferably, the expression level of RNA transcript of HER2 is determined first) there is no misclassification of HER2-positive into luminal A and B subtypes, a problem that often occurs if the subtyping is based on hierarchal cluster analysis or correlation analysis (Perou et al. (2000), Nature, 406:747-752; TCGA (Cancer Genome Atlas Network) (2012), Nature, 490:61-70). Furthermore, there is no misclassification of false-positive and clinically HER2-negative tumors, which, according to Perou et al., may occur in up to 30% of these cases.

The present invention provides methods and kits to determine hormone receptor status of ESR1 and PGR that facilitates direct decision making in favor of or against adjuvant/neoadjuvant endocrine therapy in bladder cancer due to the distinction of luminal versus non-luminal subtypes by reliable detection of ESR1 and PGR RNA transcript. This is not possible with immunohistochemical reagents due to lack of sensitivity of the IHC method and the comparably lower ESR1 and PGR expression in bladder cancer compared to, e.g., breast cancer. Median mRNA expression of ESR1 and PGR in male NMIBC (ESR1 33,96; PGR 30,96) was lower than that observed in early breast cancer of women (ESR1 40,14; PGR 36,37). This explains, why the IHC method, which is already in breast cancer at its limit of detection, is not appropriate for bladder tumor characterization. Importantly, in breast cancer, 1% hormone receptor-positive cells define the tumor to be completely hormone receptor-positive and depict tumors that are sensitive to endocrine treatment, which is clinically not possible, if 99% of the tumor cells, which look "negative" based on IHC, would indeed be hormone receptor-negative.

The methods and kits of the present invention also facilitate direct decision-making in favor of or against adjuvant/neoadjuvant chemotherapy in bladder cancer due to the distinction of luminal A and luminal B subtypes by reliable detection of ESR1, PGR and Ki67 RNA transcript. Luminal B tumors harbor a higher risk of disease-specific death and are, therefore, undertreated by transurethral resection and BCG-instillation therapy.

The methods and kits of the present invention also facilitate direct decision-making in favor of or against adjuvant/neoadjuvant chemotherapy in bladder cancer due to the identification of triple-negative bladder cancer with a substantially higher risk of approximately 30% of cancer-specific death. While luminal A tumors (~ 30% of all superficial bladder cancers) do have an excellent survival rate of up to 100% after 8 years, the triple-negative bladder cancer has a risk of approximately 30 to 40% for disease-specific death. For these patients, the primary treatment by transurethral resection and BCG-instillation only has set them at this elevated risk, while a chemotherapeutic treatment and/or an immediate cystectomy would have had a high probability to be effective and/or curative. Furthermore, the sensitivity of triple-negative bladder cancer towards platinum-based chemotherapeutic regimens justifies performing neoadjuvant chemotherapy and thereby offers the possibility of achieving pathologically complete responses with excellent long-term survival associated with less radical surgeries.

The methods and kits of the present invention further facilitate direct decision-making in favor of or against anti-HER2 treatment with or without adjuvant/neoadjuvant chemotherapy or anti-HER2 regimen being combined with chemotherapy agents in bladder cancer due to the determination of HER2 RNA transcript. The treatment with anti-HER2 regimen combined with chemotherapeutic agents (e.g. by using conjugates of anti-HER2 antibodies and chemotherapeutic agents) is particularly attractive in superficial bladder cancer, since chemotherapy is currently not indicated in this treatment situation with the patients being frequently older with comorbidities, frequently of the heart.

The present invention allows a distinction of luminal from triple-negative bladder cancer with clearly differing disease-specific survival rates. While luminal B patients have a residing risk of disease-specific death, the risk of luminal A patients is substantially lower (if present at all, even when performing transurethral resection and BCG-instillation only, i.e. without chemotherapy and/or endocrine treatment). Thus, the methods and kits also allow for the assessment of a patient's risk of disease-specific death.

Novelties of the present invention include not only the mRNA-based determination of breast cancer biomarkers in bladder cancer, but also the algorithmic definition of the subtypes. The inclusion of PGR-positivity for the definition of the luminal A subtype irrespective of ESR1-positivity enlarges the luminal A subtype group exhibiting up to 100% disease-specific survival after 8 years.

All these new aspects of the present invention contribute to the prognostic value of the RT-qPCR-based approach and kit of the present invention. In fact, these novelties provide a more accurate and meaningful subtyping of patients particularly in the non-muscle-invasive stage, which ultimately provides a prognostic tool for more individualized treatment decisions in bladder cancer.

Taken together, the present invention serves currently largely unmet clinical needs for a reliable, reproducible and quantitative assessment of prognosis and prediction of therapy success in at least 70% of all bladder cancer patients.

The present invention is further illustrated by the following examples which are not be construed as limiting the scope of the invention.

### EXAMPLES

### Example 1: Determination of mRNA expression levels by reverse transcription (RT) quantitative PCR (RT-qPCR)

RNA was isolated from formalin-fixed paraffin-embedded tissues (= FFPE tissues). More particularly, total RNA from a 5 to 10 µm curl of FFPE tumor tissue was extracted using the RNXtract^{®} Extraction Kit (BioNTech Diagnostics GmbH, Mainz, Germany) and qualified by real-time fluorescence RT-qPCR of a fragment of the reference gene CALM2. In general, 2,5 µl RNA of each qualified extraction (approx. 50-100 ng) were assayed by RT-qPCR as described below.

For a detailed analysis of gene expression by RT-qPCR methods, primers flanking the region of interest and a fluorescently labeled probe hybridizing in-between were utilized. Target-specific primers and probes were selected using the NCBI primer designing tool (www.ncbi.nlm.nih.go). RNA-specific primer/probe sequences were used to enable RNA-specific measurements by locating primer/probe sequences across exon/exon boundaries. Furthermore, primers/probes were selected not to bind to sequence regions with known polymorphisms (SNPs). In case multiple isoforms of the same gene existed, primers were selected to amplify all relevant splice variants. All primer pairs were checked for specificity by conventional PCR reactions. After further optimization of the primers/probes, the primers and probes listed in **Table 1** gave the best results. These primers/probes are superior to primers/probes known from the prior art, e.g., in terms of specificity and amplification efficiency. To standardize the amount of sample RNA, the genes CALM2 and B2M were selected as reference genes, since they were not differentially regulated in the samples analyzed.

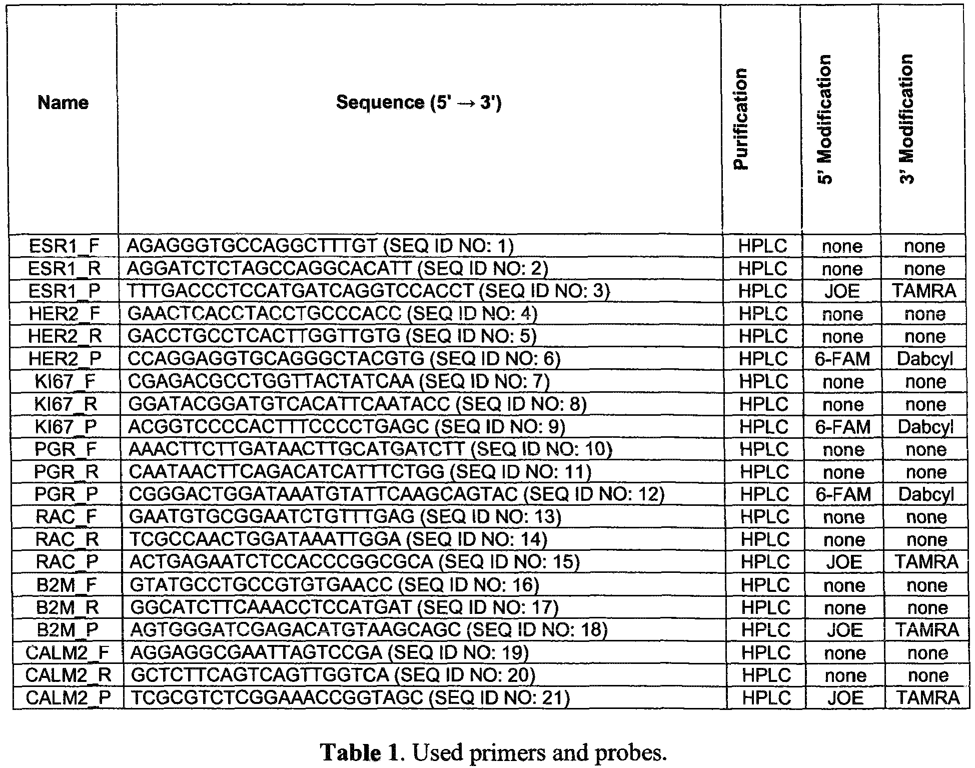

TaqMan^{®} validation experiments were performed showing that the efficiencies of the target and the control amplifications were approximately equal, which is a prerequisite for the relative quantification of gene expression by the comparative ΔCT method. To perform the expression analysis of genes of interest within a biological sample, 4x duplex assay-mixtures were prepared by mixing the respective primers/probes of two specific assays. For separate detection of CT values, the assay probes were modified with different fluorescent probes. Each 4x assay-mix contained 2 µM of unmodified forward and reverse primers and 1,2 µM of probe. For each reaction, 2,5 µl total RNA extracted from FFPE sections (see above) were mixed with 2,5 µl assay-mix, 2,5 µl enzyme-mix and 2,5 µl water in one well of a 96-well-optical reaction plate. Measurements of the PCR reaction were done according to the instructions of the manufacturer with a Versant kPCR Cycler (Siemens) or a Light Cycler 480 (Roche) under appropriate conditions (5 min 50°C, 1 cycle; 20 s 95°C, 1 cycle; 15 s 95°C; 1 min 60°C, 40 cycles). Prior to the measurement of so far unclassified biological samples, control experiments with, e.g., cell lines, healthy control samples, samples of defined molecular tumor subtypes can be used for standardization of the experimental conditions.

### Example 2: Molecular subtyping of NMIBC tumors based on the mRNA expression levels of HER2, ESR1, PGR and Ki67

For the purpose of present invention, FFPE tissues of 82 patients with NMIBC at stage pT1 treated by standard therapy were analyzed. Total RNA was isolated by commercial RNA extraction kits and mRNA expression was measured by a one-step RT-qPCR using RNA-specific TaqMan^{®} assays. Relative gene expression was determined by normalization to two housekeeping genes (CALM2, B2M) and using synthetic *in vitro* transcripts of target and housekeeping genes as control using the 40-ΔΔCT method. Prognostic value of ESR1 and PGR mRNA expression was analyzed by unsupervised cluster analysis, partitioning tests, Mann Whitney tests and Kaplan Meier estimates of cancer-specific survival (CSS). Median follow-up was 62 months.

The mean cut-off values (given as 40-ΔΔCT values) were as follows: HER2: 40.9; ESR1: 35.12; PGR: 31.24; Ki67: 36.12. The cut-off values for HER2 were determined based on evaluation of previous breast cancer studies (Koutras et al. (2008), Brit. J. of Canc., 99:1775-1785; Pentheroudakis et al. (2009), Breast Cancer Res Treat 2009, 116:131-143) using 268 samples and as applied to the FinHER study (proportional hazards model validation). CALM2 served as reference gene.

Based on the mRNA expression level of HER2, ESR1, PGR and Ki67 (neg/low indicates an expression level which is lower than the defined expression threshold; pos/increased indicates an expression level which is higher than the defined expression threshold) tumors are allocated to the molecular subtypes HER2-positive (HER2+), luminal A (LumA), luminal B (LumB) and triple-negative (TNT). As shown in **Table 2,** the molecular subtyping of tumors in accordance with the present invention differs from those that are based on prior art methods for breast cancer, e.g., by immunohistochemistry (Goldhirsch et al. (2011), Annals of Oncology, 22:1736-1747, St. Gallen International Expert Consensus on the Primary Therapy of Early Breast Cancer 2011) and analysis of only three marker genes (Sotiriou et al. (2009), N Engl J Med, 360(8):790-800).

**Table 2. Molecular subtyping of tumors.**

| **HER2** | **ESR1** | **PGR** | **Ki67** | **Present invention for bladder cancer** | **Goldhirsch et al. (2011) for breast cancer based on IHC** | **Sotiriou et al. (2009) for breast cancer based on IHC** |
|---|---|---|---|---|---|---|
| neg | neg | neg | low | TNT | TNT | basal-like |
| neg | neg | neg | increased | TNT | TNT | basal-like |
| neg | pos | pos | low | LumA | LumA | LumA |
| neg | neg | pos | low | LumA | LumA | LumB |
| neg | neg | pos | increased | LumB | LumB | LumB |
| neg | pos | neg | low | LumB | LumA | LumB |
| neg | pos | pos | increased | LumB | LumB | LumB |
| neg | pos | neg | increased | LumB | LumB | LumB |
| pos | pos | pos | increased | HER2+ | LumB | HER2+ |
| pos | pos | pos | low | HER2+ | LumB | HER2+ |
| pos | pos | neg | low | HER2+ | LumB | HER2+ |
| pos | neg | neg | low | HER2+ | HER2+ | HER2+ |
| pos | neg | neg | increased | HER2+ | HER2+ | HER2+ |

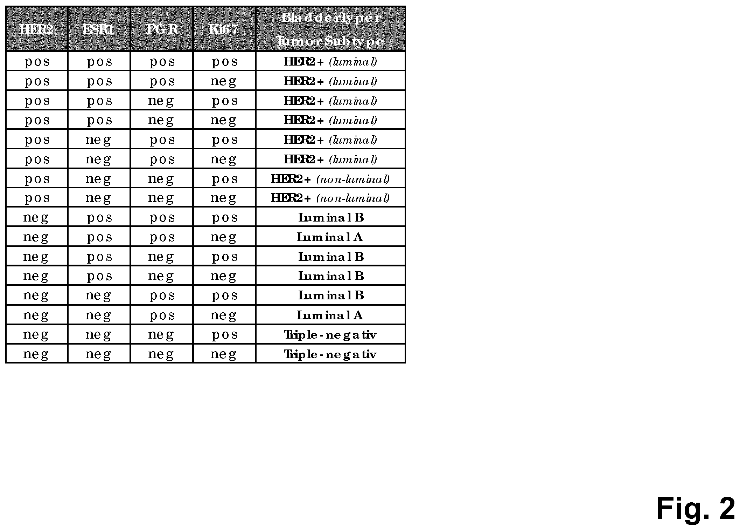

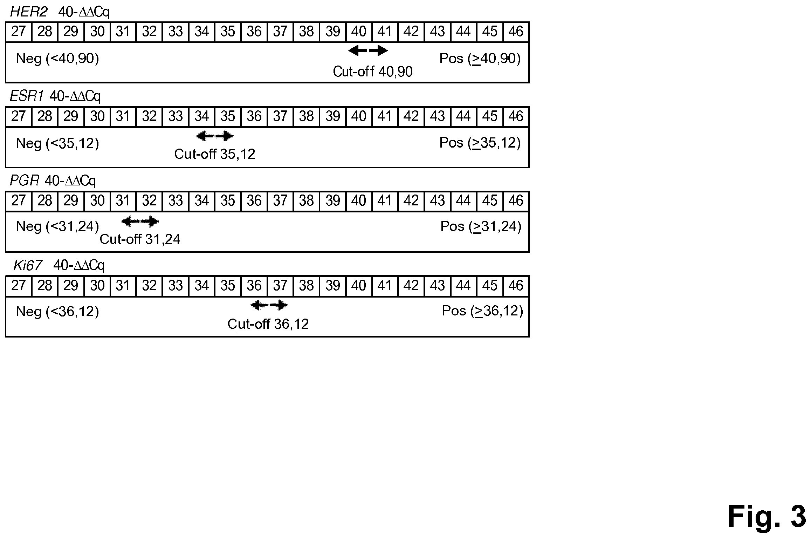

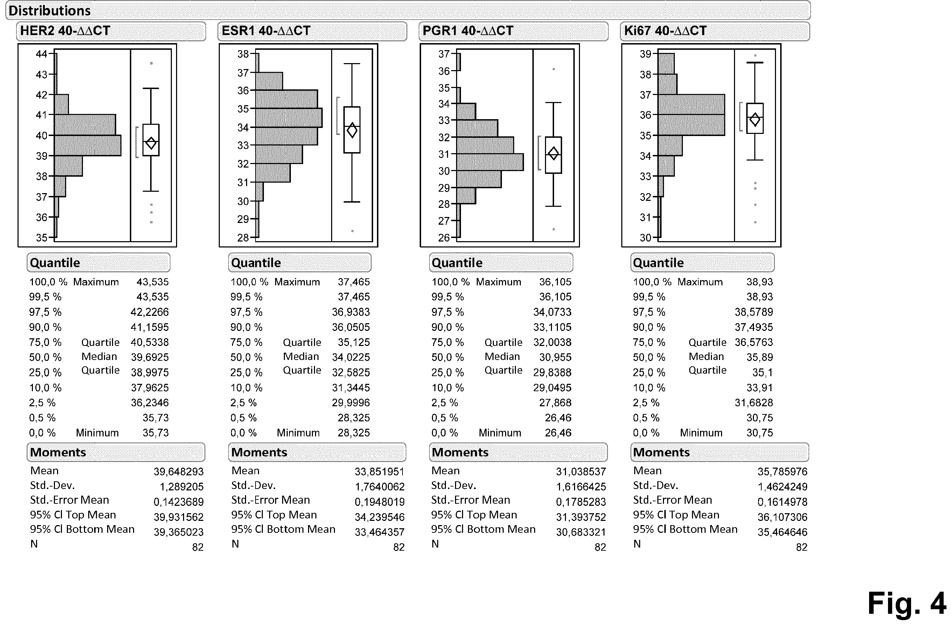

**Figure 2** shows the classification scheme of the present invention and **Figure 3** the used cut-off values within the quantitative continuous expression levels of the individual markers. **Figure 4** depicts the frequency of the expression levels as being found in a non-muscle-invasive bladder cancer cohort when determined with the BladderTyper RT-qPCR kit as described herein.

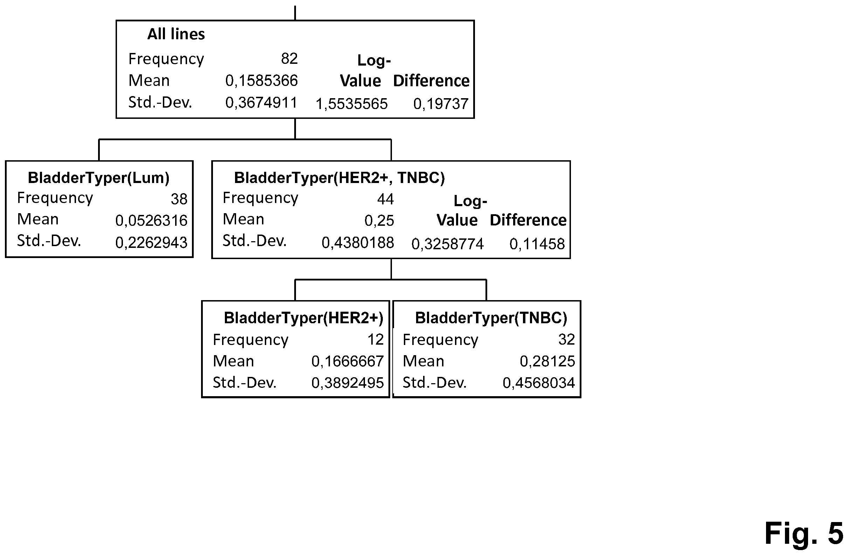

**Figure 5** depicts by partitioning the distinction and the size of the subgroups of luminal (luminal A/B; Lum), HER2-positive and triple-negative non-muscle-invasive bladder cancer. 40% of the tumors are triple-negative bladder cancer, 15% are HER2-positive and 46% are luminal tumors. The Kaplan Meier analysis depicted in **Figure 6** demonstrates that luminal tumors as defined by mRNA analysis of ESR1, PGR and HER2 have the best disease-specific survival rate (90%) after 5 years, while the disease-specific survival of HER2-positive and triple-negative bladder cancer is significantly and clinically relevantly lower (80% and 70%, respectively).

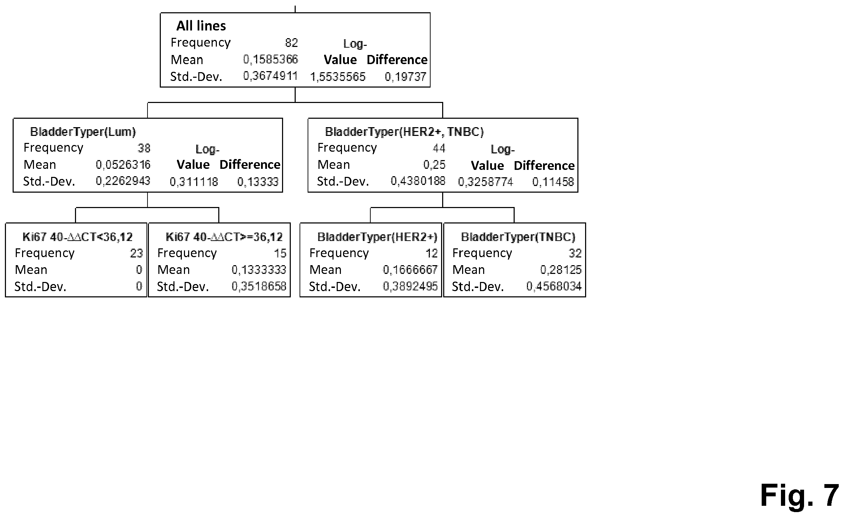

**Figure 7** depicts how the inclusion of Ki67 mRNA expression levels, when determined by the BladderTyper RT-qPCR kit as described herein, improves the outcome prediction by identifying non-muscle-invasive luminal A-type bladder cancer patients that exhibit 100% disease-specific survival 8 years after transurethral resection and BCG-instillation therapy. This luminal A subgroup comprises 28 % of all NMIBC patients of this cohort and is consistent with the expected 30% of bladder cancer patients being cured by the standard therapy. In contrast, luminal B patients comprising 18% of all NMIBCs of this cohort have a somewhat elevated risk of death due to higher tumor proliferation and resulting aggressiveness.

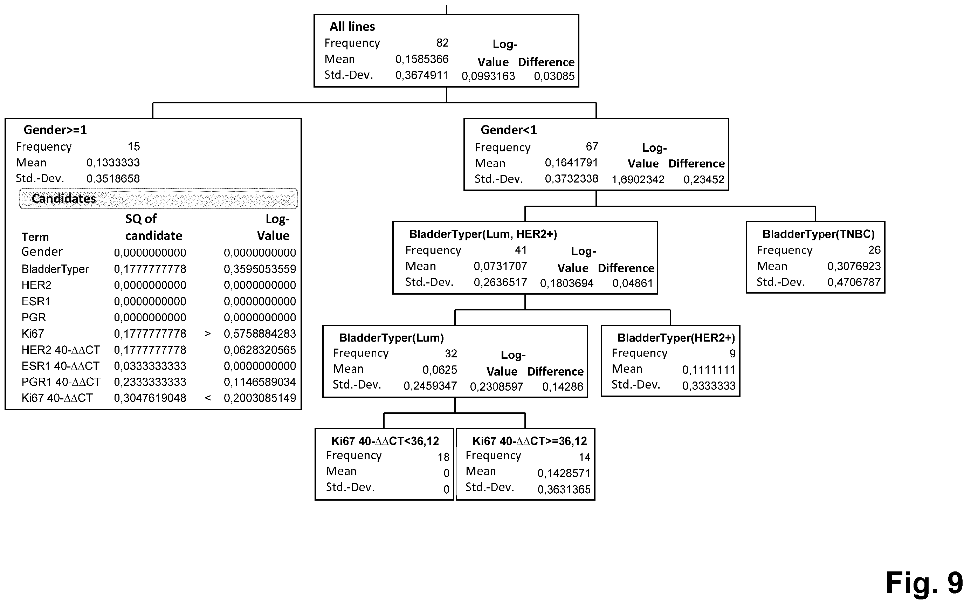

This is further shown by the Kaplan Meier analysis in **Figure 8****.** The luminal A subtype, as defined by the present invention, is associated with a disease-specific survival rate of 100% after 8 years, while luminal B patients have a 20% higher risk to die due to bladder cancer within the first 5 years after transurethral resection and BCG-instillation (without further adjuvant or neoadjuvant treatment). The disease-specific survival of HER2-positive and triple-negative patients is significantly lower (70% and 60% after 8 years, respectively). The results for male patients only are shown in **Figure 9** and **Figure 10****.**

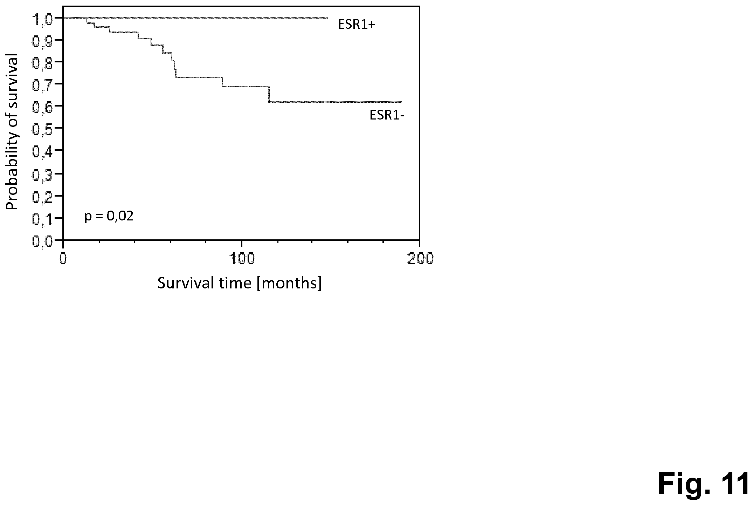

**Figure 11** depicts the prognostic relevance of ESR1 mRNA single marker determination in bladder cancer when assessed according to the methods of the present invention. ESR1-positive tumors have significantly higher disease-specific survival rates than ESR1-negative tumors (p=0,02).

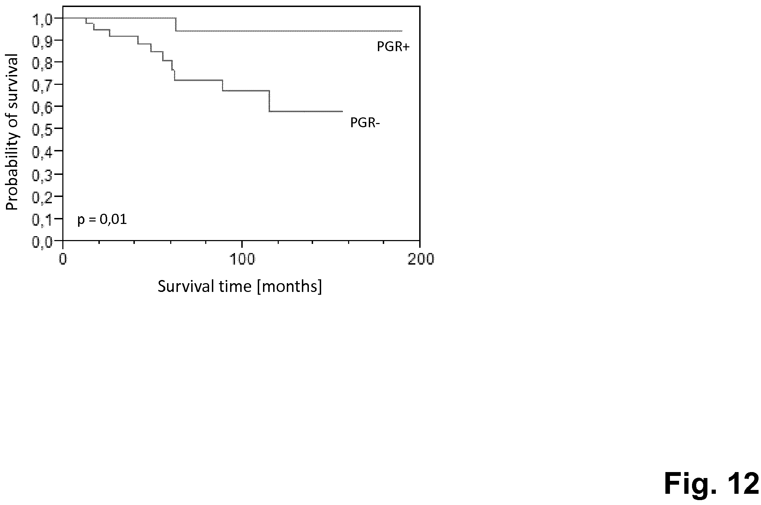

**Figure 12** depicts the prognostic relevance of PGR mRNA single marker determination in bladder cancer when assessed according to the methods of the present invention. PGR-positive tumors have significantly higher disease-specific survival rates than PGR-negative tumors (p=0,01).

### Example 3: Molecular subtyping of MIBC tumors based on the mRNA expression levels of HER2, ESR1, PGR and Ki67

mRNA expression levels of ESR1, PGR, HER2 and Ki67 using RT-qPCR in FFPE tissues from patients with muscle-invasive bladder cancer (MIBC. pT1-4; Nx) were analyzed.

Gene expression was analyzed retrospectively from FFPE tissues of 92 patients (f=22; m=70; median age: 67 [range 45-97]; pT1/2: 23, pT3: 48, pT4: 21) and measured by single step RT-qPCR using RNA-specific TaqMan^{®} assays. Relative gene expression was determined by normalization to two housekeeping genes (CALM2, B2M) and using synthetic *in vitro* transcripts of target and housekeeping genes as control using the 40-ΔΔCT method. Prognostic value of singular genes and subtyping algorithm was analyzed by unsupervised partitioning tests, Mann Whitney tests and Kaplan Meier estimates of disease-specific survival (DSS).

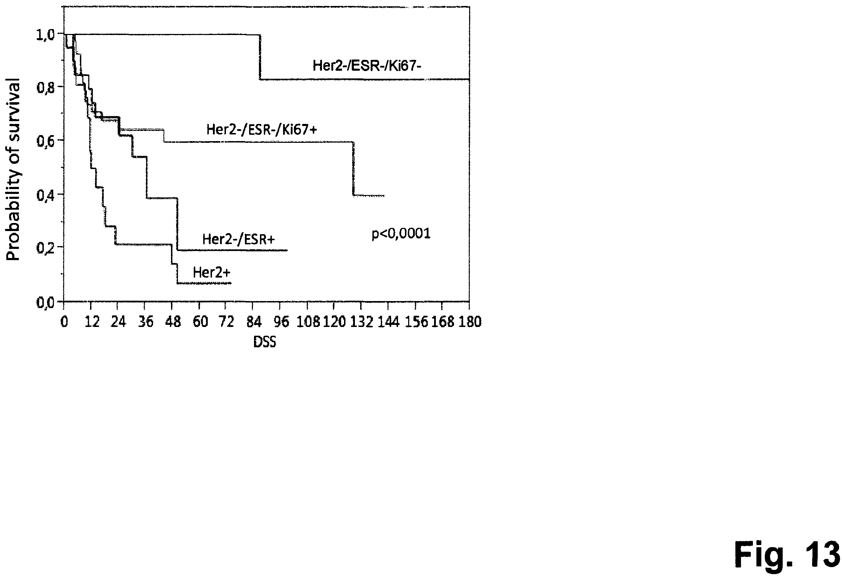

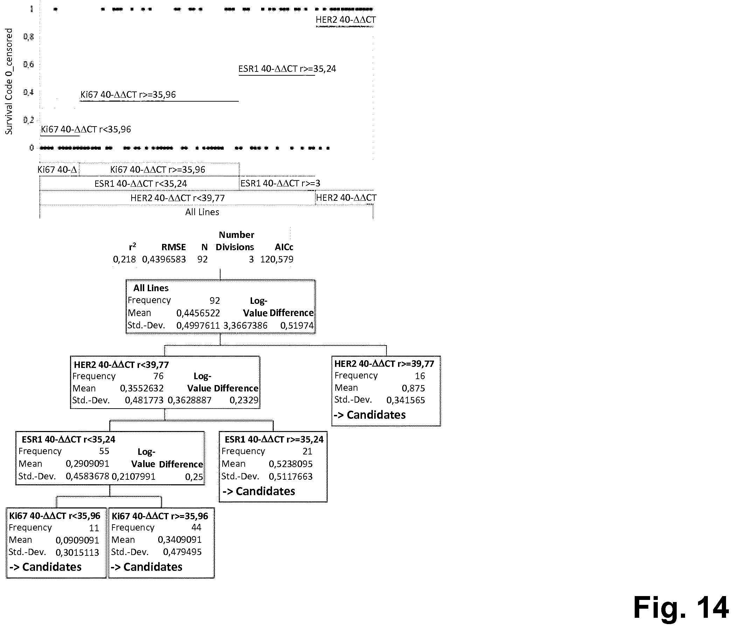

ESR1 and PGR mRNA expression was higher in comparison with HER2 und Ki67 gene expression (median ESR1 34.8; PGR: 33.9; Ki67: 37.7; HER2: 36.9). High expression of HER2 mRNA was found in 17% of patients and associated with good prognosis (57% vs 7% DSS after 5 years; p=0.0001). When combining the categorized values of ESR1, PGR, HER2 and Ki67 mRNA expression using a predefined algorithm from the breast cancer setting, the following subtypes with significantly different survival rates could be identified: HER2+ (7% DSS, after 5 years); HER2-/ESR1+ (23% of patients, 20% DSS, after 5 years); HER2-/ESR1-/Ki67+ (48% of patients, 60% DSS after 5 years); HER2-/ESR1-/Ki67- (12% of patients, 100% DSS, after 5 years; p<0,0001). The results are shown in **Figure 13** and **Figure 14****.**

These results show that subtyping by analysis of ESR1, PGR, HER2 and Ki67 mRNA levels (PGR being optional in this case) identifies molecular subtypes with prognostic relevance in MIBC. This may be helpful to identify high-risk MIBC patients and MIBC patients particularly amenable to already established targeted therapies.

### Example 4: Use of ESR1 as a prognostic marker in connection with HER2-positive NMIBC tumors

mRNA expression levels of HER2 and ESR1 using RT-qPCR in FFPE tissues from patients with non-muscle-invasive bladder cancer (NMIBC) were analyzed.

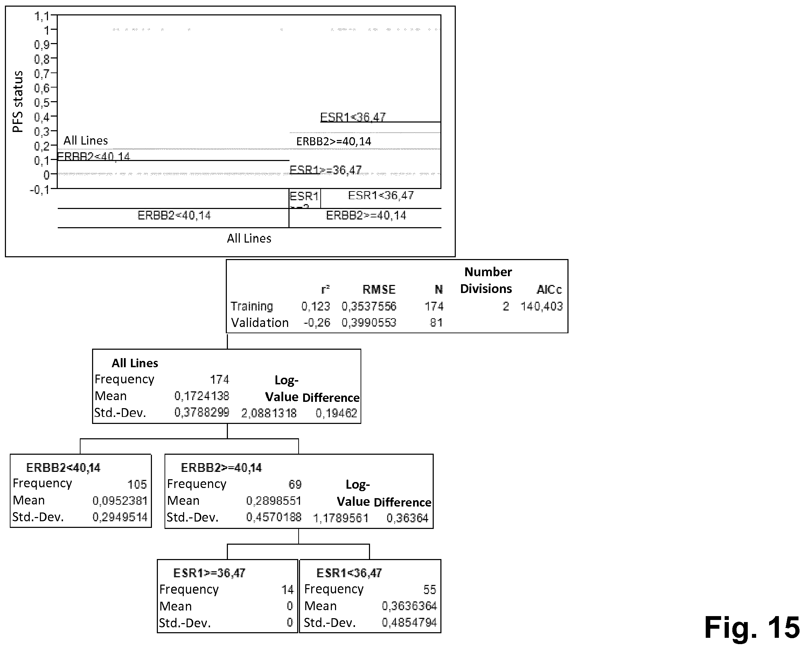

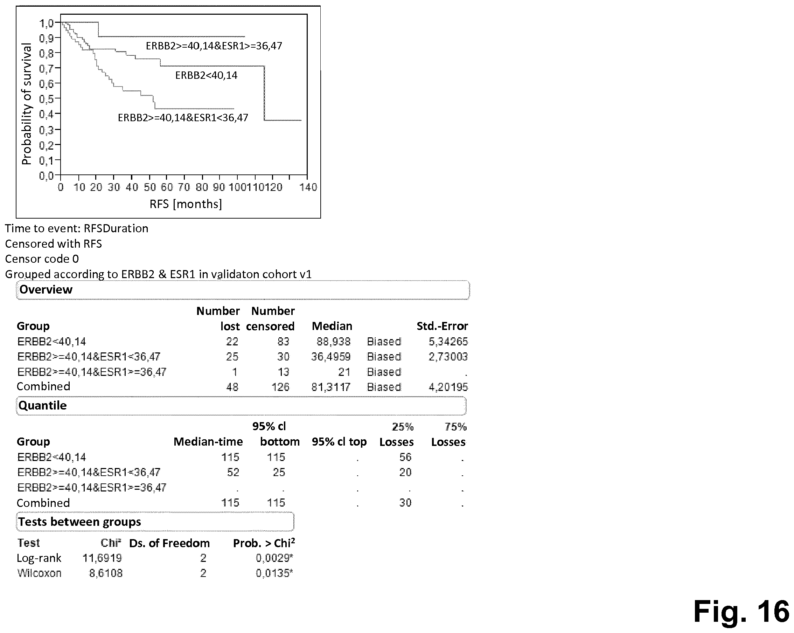

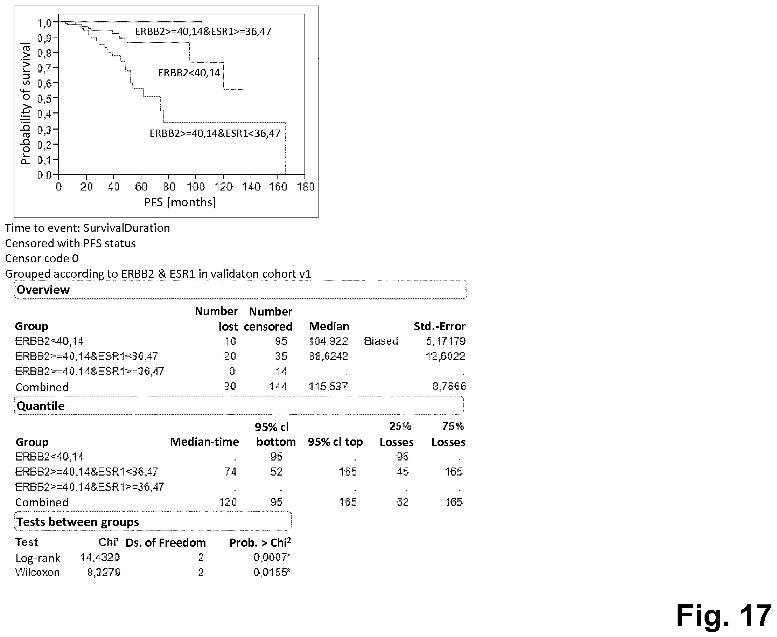

**Figures 15** to **17** show that bladder cancer patients with HER2-positive NMIBC tumors exhibit substantial differences in recurrence-free survival (RFS) and progression-free survival (PFS) depending on the concomitant mRNA expression status of ESR1. The probability of prolonged RFS and PFS is significantly higher for HER2+/ESR1+ than for HER2+/ESR1- tumor patients.

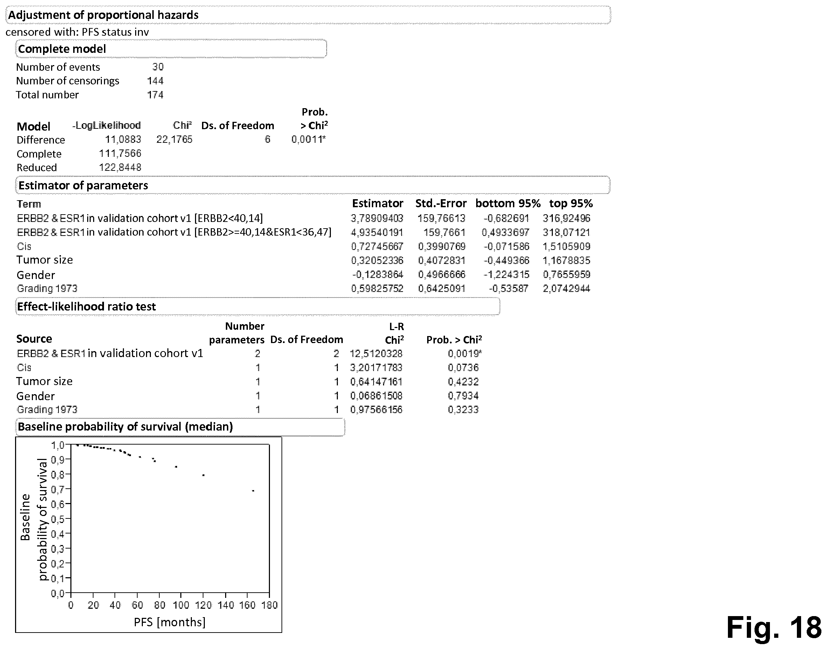

The prognostic value of ESR1 mRNA levels in HER2-positive tumors is multivariately independent of cis, grading 1973, gender and tumor size **(****Figure 18****).**

### Example 5: Molecular subtyping of NMIBC tumors - implications for recurrence-free survival (RFS) and progression-free survival (PFS)

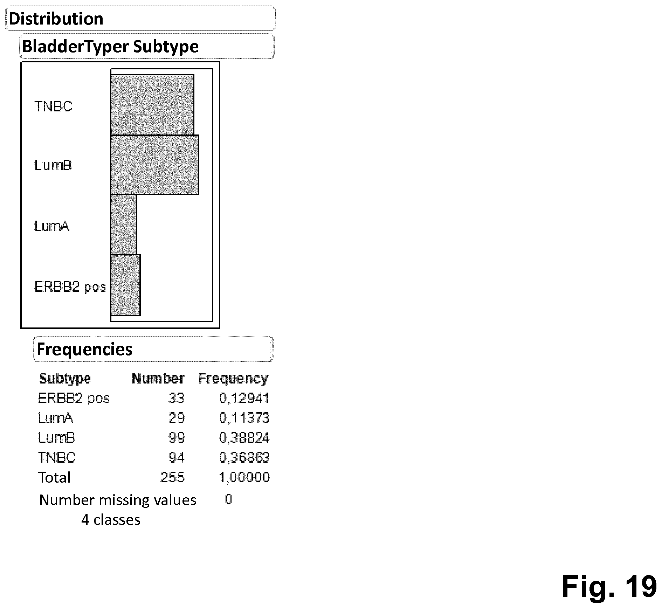

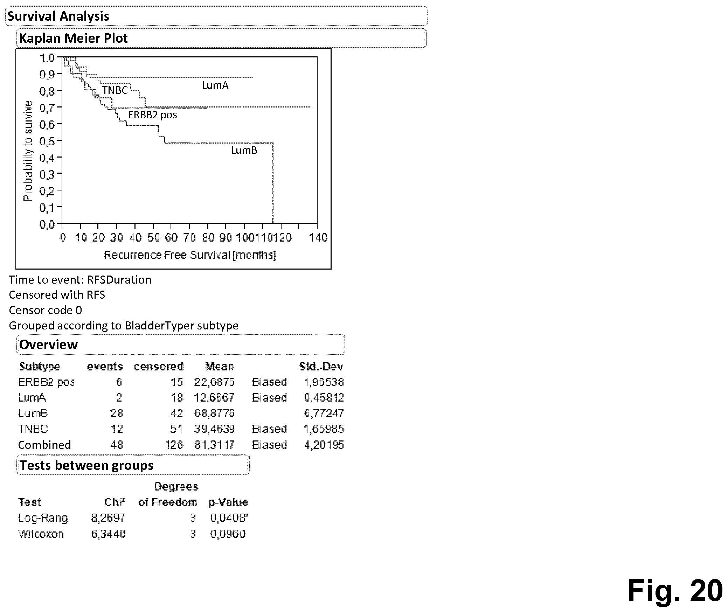

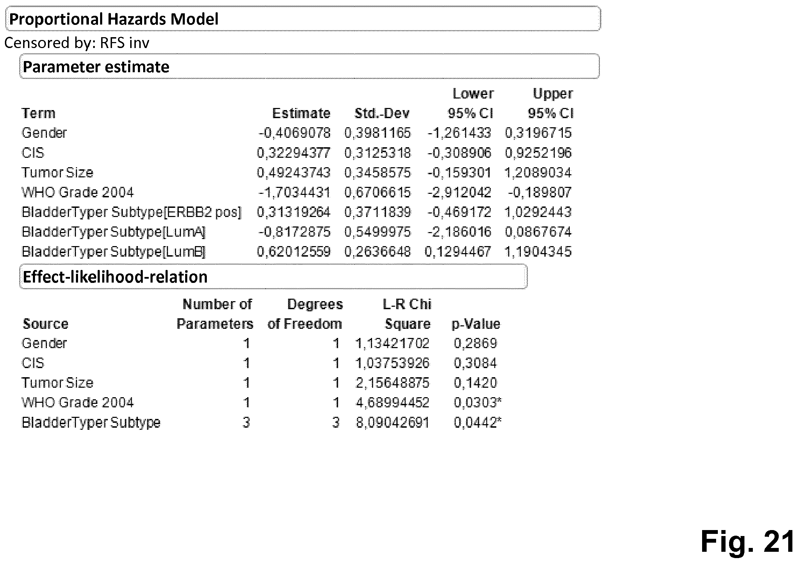

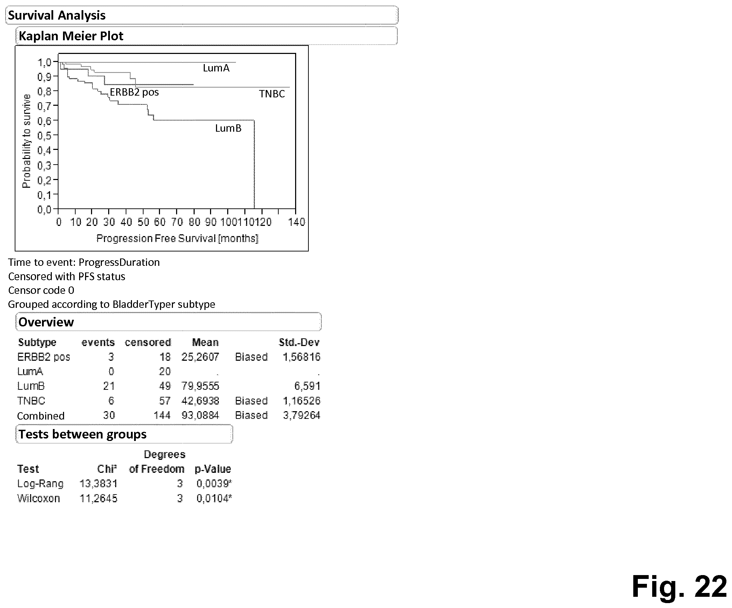

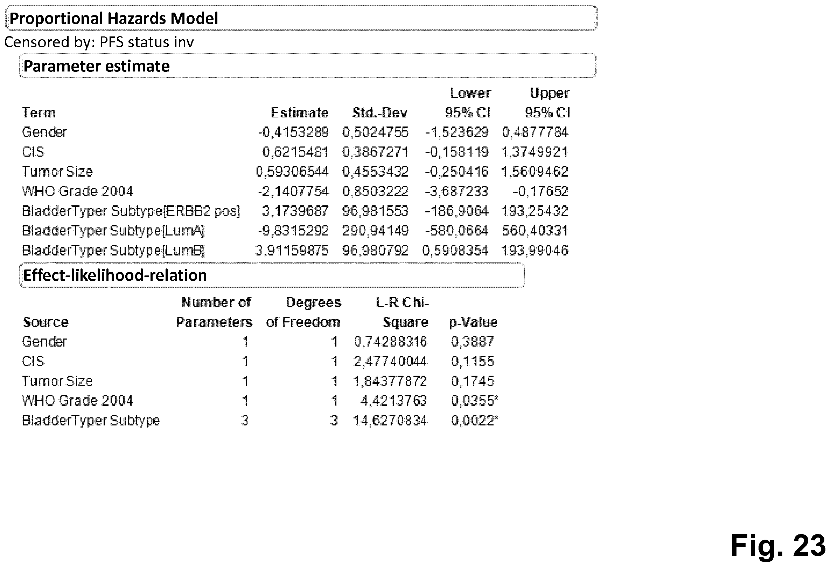

Classifying pT1 tumor samples (all technically valid samples; n=255) based on the mRNA expression levels of HER2, ESR1, PGR and Ki67 (referred to as BladderTyper technology) results in the following subtype distribution: 36,7% TNBC, 38,8% luminal B, 11,3% luminal A and 12,9% ERBB2 (HER2) positive tumors **(****Figure 19****)**. Within the validation cohort, the molecular subtyping was significant for RFS (p=0,0408) and PFS (p=0,00039) as determined by Kaplan Meier survival analysis **(****Figures 20** to **23****).** As expected, luminal A tumors had the best RFS (also referred to as disease-free survival, DFS) and PFS with no cancer-specific death in the luminal A subtype. In contrast, hormone receptor positive luminal B tumors had worse prognosis than ERBB2 positive and TNBC tumors. Therefore, by using the BladderTyper technology/algorithm, 38,8% of NMIBC can be identified as luminal B, which are at high risk for disease recurrence and tumor progress and need instillation therapy, neoadjuvant/adjuvant chemotherapy and are moreover candidates for endocrine therapy strategies, e.g., with tamoxifen (Nolvadex^{®}), fulvestrant (Faslodex^{®}) or aromatase inhibitors.

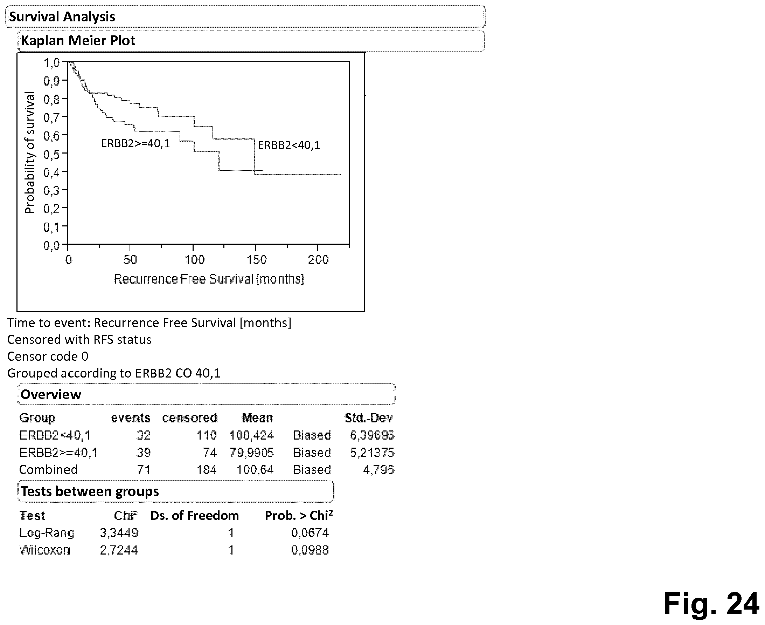

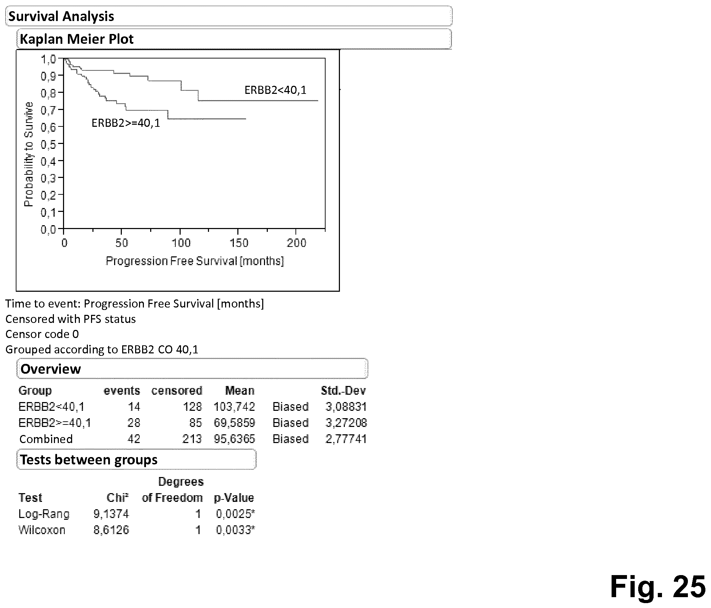

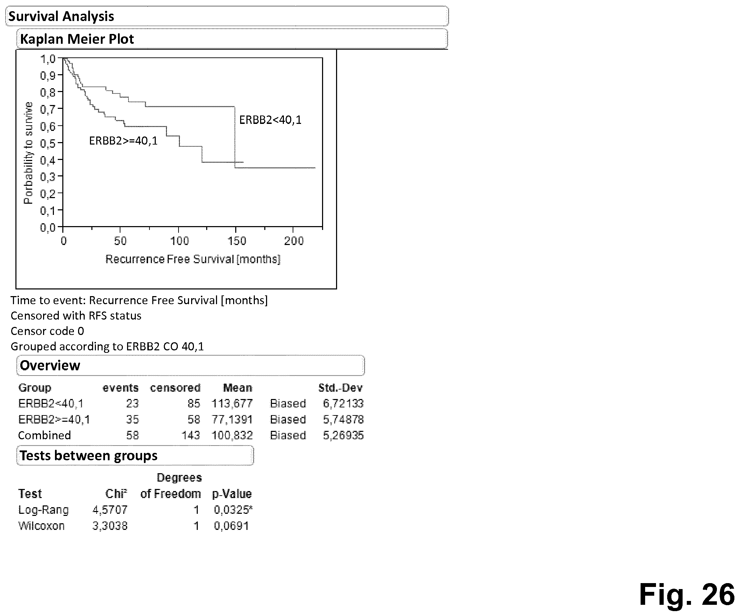

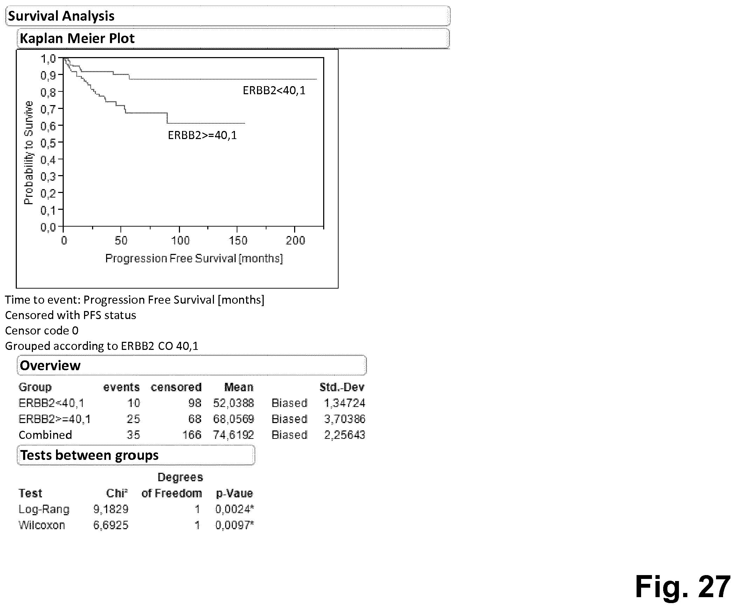

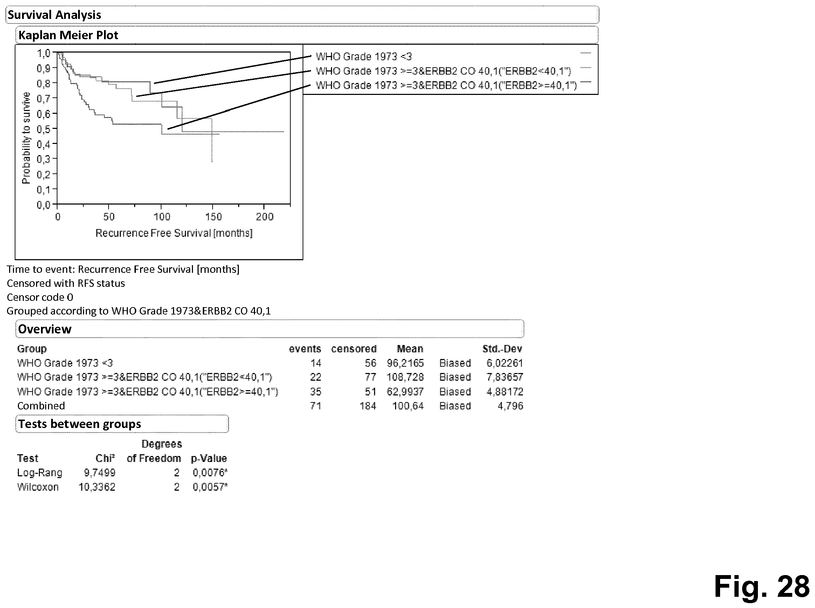

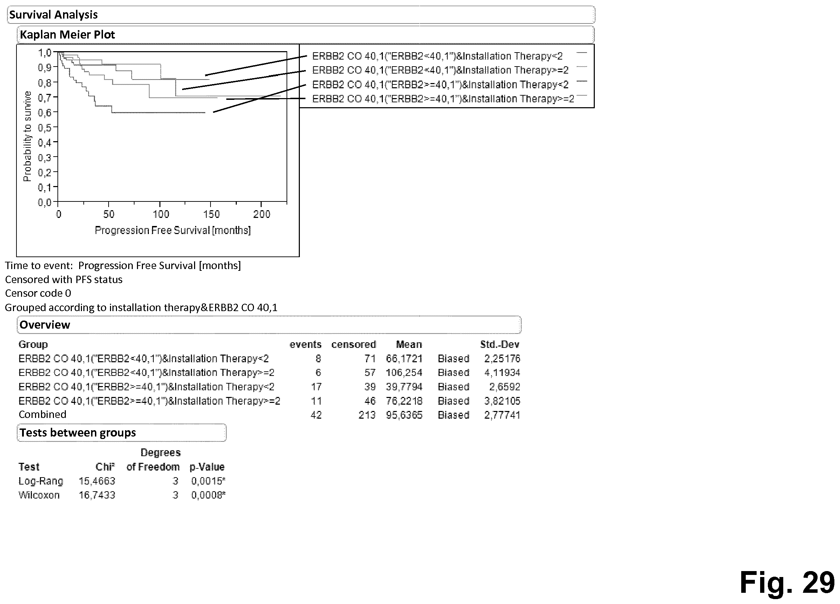

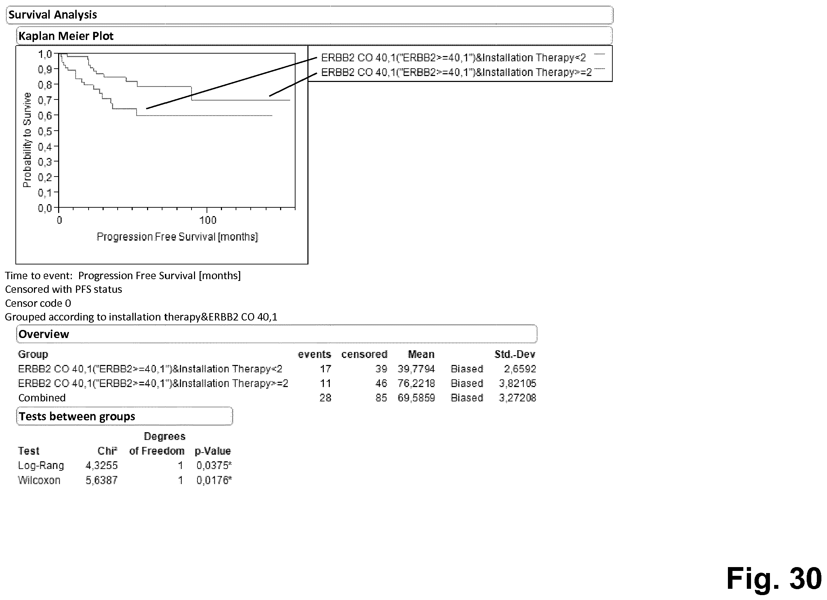

Elevated expression of HER2 above 40,1 was highly significant for PFS (p=0,0025; **Figure 25**) and trended to be significant for RFS (p=0,0674; **Figure 24**) in the total cohort - see **Figures 26** and **27** for a male patient cohort. For PFS, elevated ERBB2 mRNA expression significantly discriminated a high risk group with only 60% PFS after 5 years from a low risk group of 90% PFS after 5 year follow-up within WHO grade 3 tumors (p=0,0002) - see **Figure 28** for RFS. Interestingly, it was found that the application of instillation therapy was not effective in ERBB2 mRNA negative tumors (90% PFS versus 90% PFS after 5 years follow-up), while it provided an approximately 20% PFS survival benefit in ERBB2 mRNA positive tumors (80% PFS versus 60% PFS after 5 years follow-up; p=0,0375) - see **Figures 29** and **30****.** Therefore, mRNA based determination of elevated ERBB2 (>40,1), which cannot be identified by in situ hybridization methods or IHC methods, do benefit from instillation therapies. In contrast, tumors exhibiting lower levels of ERBB2 mRNAs do not benefit from instillation therapies and can be spared these costly, toxic and QoL affecting therapies.

### Example 6: Tumor grades/stages and mRNA expression levels of HER2, ESR1, PGR and Ki67

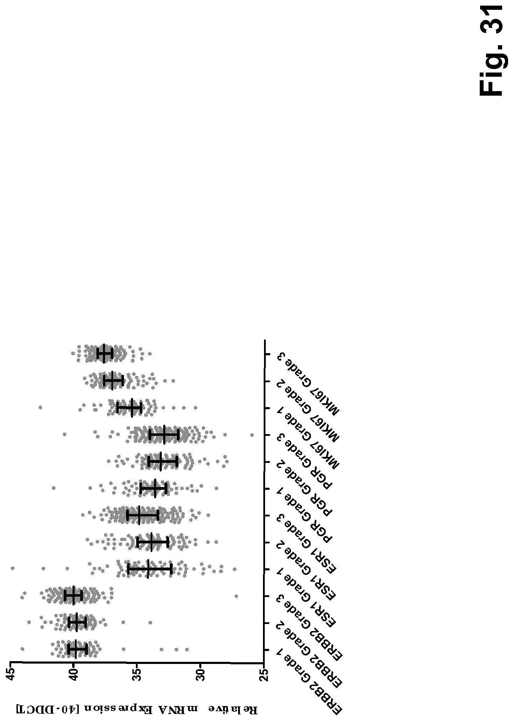

As shown in **Figure 31****,** the MKI67 (Ki67) mRNA levels differ significantly between grade 1, 2 and grade 3 tumors as determined by a non-parametric Mann-Whitney Test (p<0,0001 for each comparison). MKI67 mRNA levels also correlate well with the grade as determined by Spearman rank correlation (r=0,52; p<0,0001). In contrast, there was no significant difference for ERBB2 (HER2), ESR1 and PGR when comparing different grades. While ERBB2 and ESR1 positively correlate with grade (r=0,12; p=0,019 and r=0,15; p=0,005; respectively), PGR correlates negatively with grade (r=-0,16; p=0,0003). Based on Mann-Whitney, testing the discrimination between grade 2 and 3 tumors is significant for ERBB2 (p=0,027) and ESR1 (p=0,0006), while PGR levels differ significantly between grade 1 and grade 2 tumors (p=0,0043).

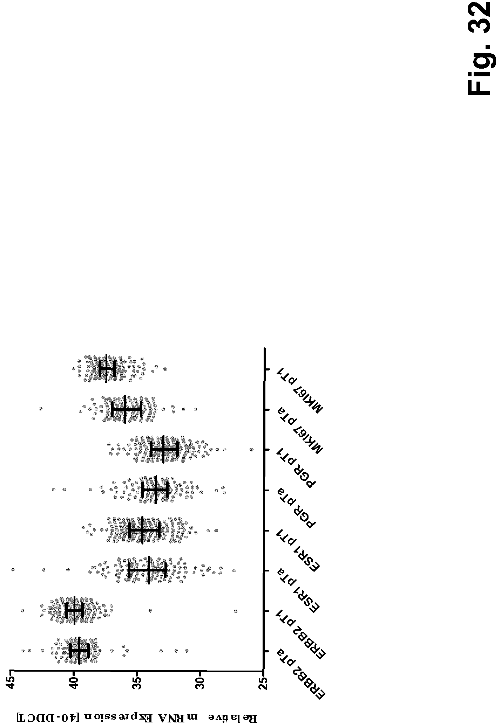

63,1% of non-muscle invasive bladder tumors exhibiting MKI67 (Ki67) mRNA levels below the cut-off value are classified as pTa **(****Figure 32****).** The MKI67 mRNA levels are significantly higher in pT1 than in pTa tumors as determined by non-parametric Mann-Whitney Test (p<0,0001). MKI67 mRNA levels also correlate well with stage as determined by Spearman rank correlation (r=0,49; p<0,0001). In contrast hereto, there is no significant difference for ERBB2 (HER2), ESR1 and PGR when comparing different tumor stages. While ERBB2 positively correlates with higher stage (r=0,16; p=0,0028), PGR correlates negatively with higher stage (r=- 0,17; p=0,00 11). Based on non-parametric Mann-Whitney testing, pTa and pT1 tumors differ significantly for ERBB2 (p=0,0058) and PGR (p=0,0007).

## Claims

1. An *in vitro* method of identifying a molecular subtype of a tumor in a patient having bladder cancer, the method comprising determining the expression level of RNA transcript of the genes human epidermal growth factor receptor 2 (HER2), estrogen receptor (ESR1), progesterone receptor (PGR), and proliferation antigen Ki-67 (Ki67) in a sample of the tumor.

2. The method of claim 1, further comprising determining the expression level of RNA transcript of RacGTPase-activating protein 1 (RACGAP1).

3. The method of claim 1 or 2, wherein the molecular subtype is selected from the group comprising HER2-positive, triple-negative, luminal A and luminal B.

4. The method of any of claims 1 to 3, wherein the bladder cancer is non-muscle-invasive bladder cancer (NMIBC) or muscle-invasive bladder cancer (MIBC).

5. The method of any of the preceding claims, wherein the sample is RNA extracted from the tumor.

6. The method of any of the preceding claims, wherein the expression level of RNA transcript is determined by reverse transcription (RT) quantitative PCR (RT-qPCR).

7. The method of claim 6, comprising the use of ESR1-specific primers having a length of 15 to 30 nucleotides and comprising at least 10 contiguous nucleotides of the sequences of SEQ ID NOs: 1 and 2, and/or HER2-specific primers having a length of 15 to 30 nucleotides and comprising at least 10 contiguous nucleotides of the sequences of SEQ ID NOs: 4 and 5, and/or Ki67-specific primers having a length of 15 to 30 nucleotides and comprising at least 10 contiguous nucleotides of the sequences of SEQ ID NOs: 7 and 8, and/or PGR-specific primers having a length of 15 to 30 nucleotides and comprising at least 10 contiguous nucleotides of the sequences of SEQ ID NOs: 10 and 11, and/or RACGAP 1-specific primers having a length of 15 to 30 nucleotides and comprising at least 10 contiguous nucleotides of the sequences of SEQ ID NOs: 13 and 14.

8. The method of claim 6 or 7, comprising the use of an ESR1-specific probe having a length of 20 to 35 nucleotides and comprising at least 15 contiguous nucleotides of the sequence of SEQ ID NO: 3, and/or a HER2-specific probe having a length of 20 to 35 nucleotides and comprising at least 15 contiguous nucleotides of the sequence of SEQ ID NO: 6, and/or a Ki67-specific probe having a length of 20 to 35 nucleotides and comprising at least 15 contiguous nucleotides of the sequence of SEQ ID NO: 9, and/or a PGR-specific probe having a length of 20 to 35 nucleotides and comprising at least 15 contiguous nucleotides of the sequence of SEQ ID NO: 12.

9. The method of claim 7 or 8, comprising the use of a RACGAP 1-specific probe having a length of 20 to 35 nucleotides and comprising at least 15 contiguous nucleotides of the sequence of SEQ ID NO: 15.

10. The method of any one of claims 1 to 9, wherein the expression level is normalized against the (mean) expression level of one or more reference genes in the sample of the tumor, wherein the one or more reference genes are preferably selected from the group comprising CALM2, B2M, RPL37A, GUSB, HPRT1 and GAPDH.

11. A method of stratifying a bladder cancer patient for tumor treatment, said method comprising, as a first step, identifying a molecular subtype of a tumor in the bladder cancer patient using the in vitro method of any one of claims 1 to 10 and, as a second step, selecting a tumor treatment regimen based on the molecular subtype identified by *the in vitro* method.

12. The method of claim 11, wherein the molecular subtype is selected from the group comprising HER2-positive, triple-negative, luminal A and luminal B.

13. The method of claim 11 or 12, wherein the bladder cancer is non-muscle-invasive bladder cancer (NMIBC) or muscle-invasive bladder cancer (MIBC).

14. The method of any of claims 11 to 13, wherein
the molecular subtype is luminal A, the bladder cancer is NMIBC, and the tumor treatment regimen comprises transurethral resection (TUR) and/or Bacillus Calmette-Guerin (BCG)-instillation, preferably TUR and BCG-instillation;
the molecular subtype is luminal B, the bladder cancer is NMIBC, and the tumor treatment regimen comprises adjuvant chemotherapy with or without adjuvant endocrine therapy;
the molecular subtype is HER2-positive, the bladder cancer is NMIBC, and the tumor treatment regimen comprises (neo)adjuvant chemotherapy with or without (neo)adjuvant anti- HER2 therapy;
the molecular subtype is triple-negative, the bladder cancer is NMIBC, and the tumor treatment regimen comprises neoadjuvant chemotherapy;
the molecular subtype is luminal A, the bladder cancer is MIBC, and the tumor treatment regimen comprises (i) cystectomy and (ii) adjuvant chemotherapy and/or adjuvant endocrine therapy, preferably adjuvant chemotherapy or adjuvant endocrine therapy;
the molecular subtype is luminal B, the bladder cancer is MIBC, and the tumor treatment regimen comprises (i) cystectomy and (ii) adjuvant chemotherapy and/or adjuvant endocrine therapy, preferably adjuvant chemotherapy and adjuvant endocrine therapy;
the molecular subtype is HER2-positive, the bladder cancer is MIBC, and the tumor treatment regimen comprises (i) cystectomy and (ii) adjuvant chemotherapy and/or adjuvant anti- HER2 therapy, preferably adjuvant chemotherapy and adjuvant anti-HER2 therapy; and/or
the molecular subtype is triple-negative, the bladder cancer is MIBC, and the tumor treatment regimen comprises neoadjuvant chemotherapy with or without subsequent cystectomy.

15. The use of a kit for identifying a molecular subtype of a tumor in a bladder cancer patient by means of reverse transcription (RT) quantitative PCR (RT-qPCR), the kit comprising a pair of primers and a probe that are specific for each of the genes HER2, ESR1, PGR, and Ki67.

16. The use of a kit of claim 15, wherein the kit further comprises a pair of primers and a probe specific for the gene RACGAP1.

## Patentansprüche

1. In-vitro-Verfahren zur Identifizierung eines molekularen Subtyps eines Tumors bei einem Patienten mit Blasenkrebs, wobei das Verfahren die Bestimmung des Expressionslevels des RNA-Transkripts der Gene humaner epidermaler Wachstumsfaktor-Rezeptor 2 (HER2), Östrogenrezeptor (ESR1), Progesteronrezeptor (PGR) und Proliferationsantigen Ki-67 (Ki67) in einer Probe des Tumors aufweist.

2. Verfahren nach Anspruch 1, ferner aufweisend die Bestimmung des Expressionslevels des RNA-Transkripts von RacGTPase-aktivierendem Protein 1 (RACGAP1).

3. Verfahren nach Anspruch 1 oder 2, wobei der molekulare Subtyp ausgewählt ist aus der Gruppe bestehend aus HER2-positiv, triple-negativ, luminal A und luminal B.

4. Verfahren nach einem der Ansprüche 1 bis 3, wobei der Blasenkrebs nicht-muskelinvasiver Blasenkrebs (NMIBC) oder muskelinvasiver Blasenkrebs (MIBC) ist.

5. Verfahren nach einem der vorhergehenden Ansprüche, wobei die Probe RNA ist, die aus dem Tumor extrahiert wurde.

6. Verfahren nach einem der vorhergehenden Ansprüche, wobei das Expressionslevel des RNA-Transkripts durch reverse-Transkription (RT) quantitative PCR (RT-qPCR) bestimmt wird.

7. Verfahren nach Anspruch 6, aufweisend die Verwendung von ESR1-spezifischen Primern mit einer Länge von 15 bis 30 Nukleotiden und aufweisend mindestens 10 zusammenhängende Nukleotide der Sequenzen der SEQ ID Nrn.: 1 und 2, und/oder HER2-spezifischen Primern mit einer Länge von 15 bis 30 Nukleotiden und aufweisend mindestens 10 zusammenhängende Nukleotide der Sequenzen der SEQ ID Nrn: 4 und 5, und/oder Ki67-spezifische Primer mit einer Länge von 15 bis 30 Nukleotiden und aufweisend mindestens 10 zusammenhängende Nukleotide der Sequenzen der SEQ ID Nrn: 7 und 8, und/oder PGR-spezifische Primer mit einer Länge von 15 bis 30 Nukleotiden und aufweisend mindestens 10 zusammenhängende Nukleotide der Sequenzen der SEQ ID Nrn: 10 und 11, und/oder RACGAP 1-spezifische Primer mit einer Länge von 15 bis 30 Nukleotiden und aufweisend mindestens 10 zusammenhängende Nukleotide der Sequenzen der SEQ ID Nrn: 13 und 14.

8. Verfahren nach Anspruch 6 oder 7, aufweisend die Verwendung einer ESR1-spezifischen Sonde mit einer Länge von 20 bis 35 Nukleotiden und aufweisend mindestens 15 zusammenhängende Nukleotide der Sequenz von SEQ ID Nr: 3, und/oder einer HER2-spezifischen Sonde mit einer Länge von 20 bis 35 Nukleotiden und aufweisend mindestens 15 zusammenhängende Nukleotide der Sequenz von SEQ ID Nr: 6, und/oder eine Ki67-spezifische Sonde mit einer Länge von 20 bis 35 Nukleotiden und aufweisend mindestens 15 zusammenhängende Nukleotide der Sequenz von SEQ ID Nr: 9, und/oder eine PGR-spezifische Sonde mit einer Länge von 20 bis 35 Nukleotiden und aufweisend mindestens 15 zusammenhängende Nukleotide der Sequenz von SEQ ID Nr: 12.

9. Verfahren nach Anspruch 7 oder 8, aufweisend die Verwendung einer RACGAP 1-spezifischen Sonde mit einer Länge von 20 bis 35 Nukleotiden und aufweisend mindestens 15 zusammenhängende Nukleotide der Sequenz von SEQ ID Nr: 15.

10. Verfahren nach einem der Ansprüche 1 bis 9, wobei das Expressionslevel gegen das (mittlere) Expressionslevel eines oder mehrerer Referenzgene in der Probe des Tumors normalisiert wird, wobei das eine oder die mehreren Referenzgene vorzugsweise ausgewählt sind aus der Gruppe aufweisend CALM2, B2M, RPL37A, GUSB, HPRT1 und GAPDH.

11. Verfahren zum Stratifizieren eines Blasenkrebspatienten für eine Tumorbehandlung, das Verfahren aufweisend, als einen ersten Schritt, das Identifizieren eines molekularen Subtyps eines Tumors in dem Blasenkrebspatienten unter Verwendung des *in vitro*-Verfahrens nach einem der Ansprüche 1 bis 10 und, als einen zweiten Schritt, das Auswählen eines Tumorbehandlungsschemas basierend auf dem durch das *in vitro*-Verfahren identifizierten molekularen Subtyp.

12. Verfahren nach Anspruch 11, wobei der molekulare Subtyp ausgewählt ist aus der Gruppe bestehend aus HER2-positiv, triple-negativ, luminal A und luminal B.

13. Verfahren nach Anspruch 11 oder 12, wobei der Blasenkrebs nicht-muskelinvasiver Blasenkrebs (NMIBC) oder muskelinvasiver Blasenkrebs (MIBC) ist.

14. Verfahren nach einem der Ansprüche 11 bis 13, wobei
der molekulare Subtyp ist luminal A ist, der Blasenkrebs NMIBC ist und das Tumorbehandlungsschema eine transurethrale Resektion (TUR) und/oder eine Bacillus Calmette-Guerin (BCG)-Instillation, vorzugsweise TUR und BCG-Instillation, aufweist;
der molekulare Subtyp luminal B ist, der Blasenkrebs NMIBC ist, und das Tumorbehandlungsschema eine adjuvante Chemotherapie mit oder ohne adjuvante endokrine Therapie aufweist;
der molekulare Subtyp HER2-positiv ist, der Blasenkrebs NMIBC ist, und das Tumorbehandlungsschema eine (neo)adjuvante Chemotherapie mit oder ohne (neo)adjuvante Anti-HER2-Therapie aufweist;
der molekulare Subtyp triple-negativ ist, der Blasenkrebs NMIBC ist, und das Tumorbehandlungsschema eine neoadjuvante Chemotherapie aufweist;
der molekulare Subtyp luminal A ist, der Blasenkrebs MIBC ist, und das Tumorbehandlungsschema (i) Zystektomie und (ii) adjuvante Chemotherapie und/oder adjuvante endokrine Therapie, vorzugsweise adjuvante Chemotherapie oder adjuvante endokrine Therapie aufweist;
der molekulare Subtyp luminal B ist, der Blasenkrebs MIBC ist, und das Tumorbehandlungsschema (i) Zystektomie und (ii) adjuvante Chemotherapie und/oder adjuvante endokrine Therapie, vorzugsweise adjuvante Chemotherapie und adjuvante endokrine Therapie aufweist;
der molekulare Subtyp HER2-positiv ist, der Blasenkrebs MIBC ist und das Tumorbehandlungsschema (i) eine Zystektomie und (ii) eine adjuvante Chemotherapie und/oder eine adjuvante Anti-HER2-Therapie, vorzugsweise eine adjuvante Chemotherapie und eine adjuvante Anti-HER2-Therapie, aufweist; und/oder
der molekulare Subtyp dreifach negativ ist, der Blasenkrebs MIBC ist und das Tumorbehandlungsschema eine neoadjuvante Chemotherapie mit oder ohne anschließende Zystektomie aufweist.

15. Verwendung eines Kits zur Identifizierung eines molekularen Subtyps eines Tumors bei einem Blasenkrebspatienten mittels reverse-Transkription (RT) quantitativer PCR (RT-qPCR), wobei das Kit ein Primerpaar und eine Sonde aufweist, die für jedes der Gene HER2, ESR1, PGR und Ki67 spezifisch sind.

16. Verwendung eines Kits nach Anspruch 15, wobei das Kit ferner ein Primerpaar und eine Sonde aufweist, die für das Gen RACGAP1 spezifisch sind.

## Revendications

1. Procédé *in vitro* d'identification d'un sous-type moléculaire d'une tumeur chez un patient ayant un cancer de la vessie, le procédé comprenant la détermination du niveau d'expression de transcription d'ARN des gènes du récepteur 2 de facteur de croissance épidermique humain (HER2), du récepteur d'œstrogène (ESR1), du récepteur de progestérone (PGR) et de l'antigène de prolifération Ki-67 (Ki67) dans un échantillon de la tumeur.

2. Procédé selon la revendication 1, comprenant en outre la détermination du niveau d'expression de transcription d'ARN de la protéine 1 activatrice de RacGTPase (RACGAP1).

3. Procédé selon la revendication 1 ou 2, dans lequel le sous-type moléculaire est sélectionné dans le groupe comprenant HER2-positif, triple-négatif, luminal A et luminal B.

4. Procédé selon l'une quelconque des revendications 1 à 3, dans lequel le cancer de la vessie est le cancer de la vessie non invasif sur le plan musculaire (CVNIM) ou le cancer de la vessie invasif sur le plan musculaire (CVIM).

5. Procédé selon l'une quelconque des revendications précédentes, dans lequel l'échantillon est de l'ARN extrait de la tumeur.

6. Procédé selon l'une quelconque des revendications précédentes, dans lequel le niveau d'expression de transcription d'ARN est déterminé par une PCR quantitative avec transcription inverse (RT) (RT-qPCR).

7. Procédé selon la revendication 6, comprenant l'utilisation d'amorces spécifiques à ESR1 ayant une longueur de 15 à 30 nucléotides et comprenant au moins 10 nucléotides contigus des séquences de SEQ ID n° 1 et 2, et/ou des amorces spécifiques à HER2 ayant une longueur de 15 à 30 nucléotides et comprenant au moins 10 nucléotides contigus des séquences de SEQ ID n° 4 et 5, et/ou des amorces spécifiques à Ki67 ayant une longueur de 15 à 30 nucléotides et comprenant au moins 10 nucléotides contigus des séquences de SEQ ID n° 7 et 8, et/ou des amorces spécifiques à PGR ayant une longueur de 15 à 30 nucléotides et comprenant au moins 10 nucléotides contigus des séquences de SEQ ID n° 10 et 11, et/ou des amorces spécifiques à RACGAP 1 ayant une longueur de 15 à 30 nucléotides et comprenant au moins 10 nucléotides contigus des séquences de SEQ ID n° 13 et 14.

8. Procédé selon la revendication 6 ou 7, comprenant l'utilisation d'une sonde spécifique à ESR1 ayant une longueur de 20 à 35 nucléotides et comprenant au moins 15 nucléotides contigus de la séquence de SEQ ID n° 3, et/ou une sonde spécifique à HER2 ayant une longueur de 20 à 35 nucléotides et comprenant au moins 15 nucléotides contigus de la séquence de SEQ ID n° 6, et/ou une sonde spécifique à Ki67 ayant une longueur de 20 à 35 nucléotides et comprenant au moins 15 nucléotides contigus de la séquence de SEQ ID n° 9, et/ou une sonde spécifique à PGR ayant une longueur de 20 à 35 nucléotides et comprenant au moins 15 nucléotides contigus de la séquence de SEQ ID n° 12.

9. Procédé selon la revendication 7 ou 8, comprenant l'utilisation d'une sonde spécifique à RACGAP 1 ayant une longueur de 20 à 35 nucléotides et comprenant au moins 15 nucléotides contigus de la séquence de SEQ ID n° 15.

10. Procédé selon l'une quelconque des revendications 1 à 9, dans lequel le niveau d'expression est normalisé par rapport au niveau d'expression (moyen) d'un ou plusieurs gènes de référence dans l'échantillon de la tumeur, dans lesquels les un ou plusieurs gènes de référence sont de préférence sélectionnés dans le groupe comprenant CALM2, B2M, RPL37A, GUSB, HPRT1 et GAPDH.

11. Procédé de stratification d'un patient atteint d'un cancer de la vessie pour le traitement de tumeur, ledit procédé comprenant, en tant que première étape, l'identification d'un sous-type moléculaire d'une tumeur chez le patient atteint d'un cancer de la vessie en utilisant le procédé in vitro selon l'une quelconque des revendications 1 à 10 et, en tant que seconde étape, la sélection d'un régime de traitement de tumeur sur la base du sous-type moléculaire identifié par le procédé *in vitro.*

12. Procédé selon la revendication 11, dans lequel le sous-type moléculaire est sélectionné dans le groupe comprenant HER2-positif, triple-négatif, luminal A et luminal B.

13. Procédé selon la revendication 11 ou 12, dans lequel le cancer de la vessie est le cancer de la vessie non invasif sur le plan musculaire (CVNIM) ou le cancer de la vessie invasif sur le plan musculaire (CVIM).

14. Procédé selon l'une quelconque des revendications 11 à 13, dans lequel
le sous-type moléculaire est luminal A, le cancer de la vessie est CVNIM et le régime de traitement de tumeur comprend la résection transurétrale (TUR) et/ou l'instillation de Bacille de Calmette-Guerin (BCG), de préférence TUR et l'instillation de BCG ;
le sous-type moléculaire est luminal B, le cancer de la vessie est CVNIM, et le régime de traitement de tumeur comprend une chimiothérapie adjuvante avec ou sans thérapie endocrine adjuvante ;
le sous-type moléculaire est HER2-positif, le cancer de la vessie est CVNIM, et le régime de traitement de tumeur comprend une chimiothérapie (néo)adjuvante avec ou sans thérapie anti-HER2 (néo)adjuvante ;
le sous-type moléculaire est triple-négatif, le cancer de vessie est CVNIM et le régime de traitement de tumeur comprend une chimiothérapie néoadjuvante ;
le sous-type moléculaire est luminal A, le cancer de la vessie est CVIM et le régime de traitement de tumeur comprend (i) une cystectomie et (ii) une chimiothérapie adjuvante et/ou une thérapie endocrine adjuvante, de préférence une chimiothérapie adjuvante ou une thérapie endocrine adjuvante ;
le sous-type moléculaire est luminal B, le cancer de la vessie est CVIM et le régime de traitement de tumeur comprend (i) une cystectomie et (ii) une chimiothérapie adjuvante et/ou une thérapie endocrine adjuvante, de préférence une chimiothérapie adjuvante et une thérapie endocrine adjuvante ;
le sous-type moléculaire est HER2-positif, le cancer de la vessie est CVIM et le régime de traitement de tumeur comprend (i) une cystectomie et (ii) une chimiothérapie adjuvante et/ou une thérapie HER2 anti-adjuvante, de préférence une chimiothérapie adjuvante et une thérapie anti-HER2 adjuvante ; et/ou
le sous-type moléculaire est triple-négatif, le cancer de la vessie est CVIM, et le régime de traitement de tumeur comprend une chimiothérapie néoadjuvante avec ou sans cystectomie postérieure.

15. Utilisation d'un kit pour identifier un sous-type moléculaire d'une tumeur chez un patient atteint d'un cancer de la vessie au moyen d'une PCR quantitative avec transcription inverse (RT) (RT-qPCR), le kit comprenant un couple d'amorces et une sonde qui sont spécifiques à chacun des gènes HER2, ESR1, PGR et Ki67.

16. Utilisation d'un kit selon la revendication 15, dans lequel le kit comprend en outre un couple d'amorces et une sonde spécifiques au gène RACGAP1.
